# FASCICULE DE BREVET EUROPEEN

(11) **EP 0 777 738 B1**
(45) Date de publication et mention de la délivrance du brevet: **08.06.2005**
(21) Numéro de dépôt: 95925882.3
(22) Date de dépôt: 13.07.1995
(51) Int. Cl.: C12N 15/80, C12N 15/81, C12N 15/62, C12N 1/15, C12N 1/19, C07K 14/45, C07K 16/20, G01N 33/53, G01N 33/543, A61K 48/00, A61K 39/002

(54) **CASSETTE D'EXPRESSION D'UNE PROTEINE P30 DE TOXOPLASMA GONDII**
EXPRESSION KASSETTE EINES PROTEINES P30 AUS TOXOPLASMA GONDII
CONDII TOXOPLASMA P30 EXPRESSION CASSETTE

(30) Priorité: 13.07.1994 FR 9408760
(43) Date de publication de la demande: 11.06.1997
(73) Titulaire: TRANSGENE S.A., 67082 Strasbourg Cédex (FR); BIOMERIEUX, 69280 Marcy-L'Etoile (FR)
(72) Inventeur: JACOBS, Eric, 67120 Dorlisheim (FR); SILVESTRE, Nathalie, 67100 Strasbourg (FR); MOUGIN, Bruno, 69008 Lyon (FR); BISSARDON, Odette, 69007 Lyon (FR); JOLIVET, Michel, 69500 Bron (FR)
(74) Mandataire: Warcoin, Jacques
(86) Numéro de dépôt international: PCT/FR1995/000942
(87) Numéro de publication internationale: WO 1996/002654

(56) Documents cités:
- EP-A- 0 353 111
- INFECTION AND IMMUNITY, vol. 62, no. 1, Janvier 1994 WASHINGTON US, pages 203-209, KAMI KIM ET AL. 'Conformationally appropiate expression of the toxoplasma antigen SAG1 (p30) in CHO cells' cité dans la demande
- JOURNAL OF IMMUNOLOGY., vol. 134, no. 5, Mai 1985 BALTIMORE US, pages 3426-3431, LLOYD H. KASPER ET AL. 'An unexpected response to vaccination with a purified major membrane tachyzoite antigen (P30) of Toxoplasma gondii'
- JOURNAL OF IMMUNOLOGY., vol. 141, no. 10, 15 Novembre 1988 BALTIMORE US, pages 3584-3591, J. LAWRENCE BURG ET AL. 'Molecular analysis of the gene encoding the major surface antigen of Toxoplasma gondii' cité dans la demande

### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 22 58 07

      (A) NOM: BioMerieux S.A.
      (B) RUE: Chemin de l'Orme
      (C) VILLE: Marcy l'étoile
      (E) PAYS: France
      (F) CODE POSTAL: 69280
      (G) TELEPHONE: (33) 78 87 20 00
      (H) TELECOPIE: (33) 78 87 20 90
   (ii) TITRE DE L' INVENTION: Cassette d'expression d'une proteine P30 de *Toxoplasma gondii*
   (iii) NOMBRE DE SEQUENCES: 19
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 299 acides aminés
      (B) TYPE: acide aminé
      (D) CONFIGURATION: linéaire
   (ii) TYPE DE MOLECULE: protéine
   (iii) HYPOTHETIQUE: NON
   (iii) ANTI-SENS: NON
   (vi) ORIGINE:
      (A) ORGANISME: proteine P30 de *Toxoplasma gondii*
      (B) SOUCHE: souche RH
   (ix) CARACTERISTIQUE ADDITIONELLE:
      (A) NOM/CLE: Cleavage-site
      (B) EMPLACEMENT: 30..31
      (D) AUTRES RENSEIGNEMENTS: /label= secretion /note= "signal de secretion compris entre les residus 1 a 30 compris"
   (ix) CARACTERISTIQUE ADDITIONELLE:
      (A) NOM/CLE: Peptide
      (B) EMPLACEMENT: 1..30
      (D) AUTRES RENSEIGNEMENTS: /label= secretion
   (xi) DESCRIPTION DE LA SEQUENCE: SEQ ID NO: 1:
   Met Phe Pro Lys Ala Val Arg Arg Ala Val Thr Ala Gly Val Phe Ala
   1               5                   10                  15
   Ala Pro Thr Leu Met Ser Phe Leu Arg Cys Gly Val Met Ala Ser Asp
               20                  25                  30
   Pro Pro Leu Val Ala Asn Gln Val Val Thr Cys Pro Asp Lys Lys Ser
           35                  40                  45
   Thr Ala Ala Val Ile Leu Thr Pro Thr Glu Asn His Phe Thr Leu Lys
       50                  55                  60
   Cys Pro Lys Thr Ala Leu Thr Glu Pro Pro Thr Leu Ala Tyr Ser Pro
   65                  70                  75                  80
   Asn Arg Gln Ile Cys Pro Ala Gly Thr Thr Ser Ser Cys Thr Ser Lys
                   85                  90                  95
   Ala Val Thr Leu Ser Ser Leu Ile Pro Glu Ala Glu Asp Ser Trp Trp
               100                 105                 110
   Thr Gly Asp Ser Ala Ser Leu Asp Thr Ala Gly Ile Lys Leu Thr Val
           115                 120                 125
   Pro Ile Glu Lys Phe Pro Val Thr Thr Gln Thr Phe Val Val Gly Cys
       130                 135                 140
   Ile Lys Gly Asp Asp Ala Gln Ser Cys Met Val Thr Val Thr Val Gln
   145                 150                 155                 160
   Ala Arg Ala Ser Ser Val Val Asn Asn Val Ala Arg Cys Ser Tyr Gly
                   165                 170                 175
   Ala Asp Ser Thr Leu Gly Pro Val Lys Leu Ser Ala Glu Gly Pro Thr
               180                 185                 190
   Thr Met Thr Leu Val Cys Gly Lys Asp Gly Val Lys Val Pro Gln Asp
           195                 200                 205
   Asn Asn Gln Tyr Cys Ser Gly Thr Thr Leu Thr Gly Cys Asn Glu Lys
       210                 215                 220
   Ser Phe Lys Asp Ile Leu Pro Lys Leu Thr Glu Asn Pro Trp Gln Gly
   225                 230                 235                 240
   Asn Ala Ser Ser Asp Lys Gly Ala Thr Leu Thr Ile Lys Lys Glu Ala
                   245                 250                 255
   Phe Pro Ala Glu Ser Lys Ser Val Ile Ile Gly Cys Thr Gly Gly Ser
               260                 265                 270
   Pro Glu Lys His His Cys Thr Val Lys Leu Glu Phe Ala Gly Ala Ala
           275                 280                 285
   Gly Ser Ala Lys Ser Ala Ala Gly Thr Ala Ser
       290                 295
(2) INFORMATION POUR LA SEQ ID NO: 2:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthèse 1367
   (xi) DESCRIPTION DE LA SEQUENCE: SEQ ID NO: 2:
   GCATGCAAGC TTGGATAAAA AGATCGGATC CCCCTCTTG 39
(2) INFORMATION POUR LA SEQ ID NO: 3:
   (i) CARACTERISTIQUES DE LA SEQUENCE:
      (A) LONGUEUR: 29 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthèse 1366
   (xi) DESCRIPTION DE LA SEQUENCE: SEQ ID NO: 3:
   GCGTCAGTCG ACATTCAACT GGCTGTTCC 29
(2) INFORMATION POUR LA SEQ ID NO: 4:
   (i) CARACTERISTIQUES DE LA SEQUENCE:
      (A) LONGUEUR: 79 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthèse (OTG2781)
   (xi) DESCRIPTION DE LA SEQUENCE: SEQ ID NO: 4:
   AATTCCATTG TCTTGACTAT CACAAACTTT TAAGTCTTTT CTTTTTTGGA TCCACACCAT    60
   GGAGCTCCCG GGAGATCTA                                                  79
(2) INFORMATION POUR LA SEQ ID NO: 5:
   (i) CARACTERISTIQUES DE LA SEQUENCE:
      (A) LONGUEUR: 79 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthese (OTG2782)
   (xi) DESCRIPTION DE LA SEQUENCE: SEQ ID NO: 5:
   AGCTTAGATC TCCCGGGAGC TCCATGGTGT GGATCCAAAA AAGAAAAGAC TTAAAAGTTT    60
   GTGATAGTCA AGACAATGG                                                  79
(2) INFORMATION POUR LA SEQ ID NO: 6:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthèse 722
   (xi) DESCRIPTION DE LA SEQUENCE: SEQ ID NO: 6:
   TCTGGTTAGA TCTAAAAAAT GTCTCCGAAG                                      30
(2) INFORMATION POUR LA SEQ ID NO: 7:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthese 723
   (xi) DESCRIPTION DE LA SEQUENCE: SEQ ID NO: 7:
   AAAAATGAGA TCTTAACTGG CTGT 24
(2) INFORMATION POUR LA SEQ ID NO: 8:
   (i) CARACTERISTIQUES DE LA SEQUENCE:
      (A) LONGUEUR: 71 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthèse (OTG4096)
   (xi) DESCRIPTION DE LA SEQUENCE: SEQ ID NO: 8:
   GATCCAGATC TAAAATGTTC TTGCAAAATT TATTCCTTGG CTTTTTGGCC GTCGTTTGTG 60
   CCAACGCGTC G 71
(2) INFORMATION POUR LA SEQ ID NO: 9:
   (i) CARACTERISTIQUES DE LA SEQUENCE:
      (A) LONGUEUR: 71 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthèse (OTG4097)
   (xi) DESCRIPTION DE LA SEQUENCE: SEQ ID NO: 9:
   GATCCGACGC GTTGGCACAA ACGACGGCCA AAAAGCCAAG GAATAAATTT TGCAAGAACA 60
   TTTTAGATCT G 71
(2) INFORMATION POUR LA SEQ ID NO: 10:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: oligonucleotide de synthèse (OTG5829)
   (xi) DESCRIPTION DE LA SEQUENCE: SEQ ID NO: 10:
   GGCAGGGTCA AGCTTCGAG 19
(2) INFORMATION POUR LA SEQ ID NO: 11:
   (i) CARACTERISTIQUES DE LA SEQUENCE:
      (A) LONGUEUR: 74 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: oligonucleotide de synthèse (OTG2872)
   (xi) DESCRIPTION DE LA SEQUENCE: SEQ ID NO: 11:
   GATCCAACCA CATAATGTTC TTGCAAAATT TATTCCTTGG CTTTTTGGCC GTCGTTTGTG    60
   CCAACGCGTG AGCT                                                      74
(2) INFORMATION POUR LA SEQ ID NO: 12:
   (i) CARACTERISTIQUES DE LA SEQUENCE:
      (A) LONGUEUR: 66 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: oligonucleotide de synthese (OTG2873)
   (xi) DESCRIPTION DE LA SEQUENCE: SEQ ID NO: 12:
   CACGCGTTGG CACAAACGAC GGCCAAAAAG CCAAGGAATA AATTTTGCAA GAACATTATG    60
   TGGTTG                                                               66
(2) INFORMATION POUR LA SEQ ID NO: 13:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthèse (OTG3569)
   (xi) DESCRIPTION DE LA SEQUENCE: SEQ ID NO: 13:
   TTTGGGCATG CGTCTTTTGA TGCTAAATAA ATTAAATTGT TGG 43
(2) INFORMATION POUR LA SEQ ID NO: 14:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthèse (OTG3239)
   (xi) DESCRIPTION DE LA SEQUENCE: SEQ ID NO: 14:
   GAGAAATACC ACTTAACTTC ATGGATCCCG AGAAAAAACA ATG 43
(2) INFORMATION POUR LA SEQ ID NO: 15:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthèse (OTG3210)
   (xi) DESCRIPTION DE LA SEQUENCE: SEQ ID NO: 15:
   CCCAAACCAT GGCAATCAAT CCGGATATCT GCTAAC 36
(2) INFORMATION POUR LA SEQ ID NO: 16:
   (i) CARACTERISTIQUES DE LA SEQUENCE:
      (A) LONGUEUR: 58 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthese (OTG6438)
   (xi) DESCRIPTION DE LA SEQUENCE: SEQ ID NO: 16:
   GGGTCAGCAA AATCGGCTGC GGGAACAGCT AGCCACCATC ACCATCACCA CTGAACCC 58
(2) INFORMATION POUR LA SEQ ID NO: 17:
   (i) CARACTERISTIQUES DE LA SEQUENCE:
      (A) LONGUEUR: 62 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthèse (OTG6439)
   (xi) DESCRIPTION DE LA SEQUENCE: SEQ ID NO: 17:
   GGGTTCAGTG GTGATGGTGA TGGTGGCTAG CTGTTCCCGC AGCCGATTTT GCTGACCCTG 60
   CA 62
(2) INFORMATION POUR LA SEQ ID NO: 18:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthese (OTG10055)
   (xi) DESCRIPTION DE LA SEQUENCE: SEQ ID NO: 18:
   GATCATCATC ACCATCACCA TCATCAC                                    27
(2) INFORMATION POUR LA SEQ ID NO: 19:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthese (OTG10094)
   (xi) DESCRIPTION DE LA SEQUENCE: SEQ ID NO: 19:
   GATCGTGATG ATGGTGATGG TGATGAT                                    27

## Description

La présente invention a pour objet un système d'expression d'un antigène de surface de *Toxoplasma* gondii, l'antigène ainsi obtenu et son utilisation à des fins diagnostique et/ou thérapeutique.

La toxoplasmose est une maladie infectieuse causée par un parasite protozoaire *Toxoplasma gondii,* membre de la classe des Sporozoa et de l'ordre des Coccidia. *Toxoplasma gondii* est un parasite intracellulaire qui se reproduit dans une grande variété de types de cellules à l'intérieur de ses hôtes, qui sont les mammifères.

Ce parasite est un pathogène important, non seulement en médecine humaine, mais également en médecine vétérinaire, très largement répandu géographiquement.

Chez l'homme, deux formes du parasite ont été décrites : le "tachyzoïte", qui est la forme multiplicative rencontrée au cours de la phase aiguë de la maladie et le "bradyzoïte", forme résistante qui persiste enkystée dans les tissus nerveux et qui est vraisemblablement responsable de l'entretien d'une immunité durable à la réinfection.

Chez l'être humain, la toxoplasmose est le plus souvent asymptomatique et passe la plupart du temps inaperçue sans présenter de conséquences. Il existe cependant des cas pour lesquels une infection par toxoplasme ou une réactivation d'une infection acquise antérieurement peut générer des troubles graves pour les personnes dites à risque que sont les femmes enceintes et les sujets immunodéprimés ou immunosupprimés. Cet organisme a des sièges de réplication multiples. Cependant, il peut être responsable d'atteintes oculaires et cérébrales sévères lorsque son siège de réplication est les cellules du système nerveux central et les cellules du système réticuloendothélial. La femme enceinte représente un sujet à haut risque, puisqu'une infection toxoplasrnique, notamment pendant les premiers mois de la grossesse, peut être à l'origine de graves complications foetales et néonatales, si le traitement maternel n'est pas précocement entrepris et poursuivi avec assiduité. En particulier, les nouveaux nés contaminés par voie transplacentaire sont sujets à des troubles cérébraux et oculaires graves, voire mortels dans certains cas. Les malades immunodéprimés et particulièrement les sidéens sont sujets à des toxoplasmoses graves dues le plus souvent à des réactivations d'infections antérieures.

Il était donc indispensable de disposer de tests de diagnostic qui permettent de déterminer la présence du parasite notamment chez la femme enceinte, soit par détection d'anticorps éventuellement présents chez l'individu, soit par détection de la présence d'antigènes du toxoplasme chez le sujet.

HUGUES dans "Current topics in Microbiology and Immunology", Vol.120 (1985), SPRINGER Ed., pages 105-139 a répertorié un certain nombre de tests de sérodiagnotic disponibles commercialement, tels que le test de coloration de SABIN et FELDMAN, standardisé par BEVERLY et BEATTLE en 1958 et perfectionné par FELDMAN et LAMB (1966), WALDELAND (1976) et BALFOUR et al. (1982) ; le test de détection d'anticorps par immunofluorescence de REMINGTON (1968), optimisé en 1975 par KARIM et LUDLAM ; les tests d'hémagglutination ; le test ELISA pour la détection d'anticorps spécifiques du Toxoplasme, par isolement d'IgM in situ sur microplaque décrit en 1983 par WIELARRD et al..

Les différents tests mis en oeuvre reposent sur la détection d'anticorps ou d'antigènes spécifiques de la toxoplasmose. Un des points critiques consistait donc en la caractérisation des antigènes majeurs de *Toxoplasma gondii,* induisant une réponse immunitaire spécifique et susceptibles d'être utilisés dans des tests sérologiques de détection.

A cet égard, Burg, et al. (Abstract c85 J. Cell. Biochem., 1986, *1017*, 145) ont décrit l'exploitation d'une banque d'expression chez E. Coli en utilisant l'ADN complémentaire (ADNc) obtenu à partir d' ARN messagers de *Toxoplasma gondii* et l'isolement à partir de cette banque, de séquences codant pour les antigènes présents à la surface du parasite, à l'aide d'antisera polyclonaux dirigés contre les protéines antigéniques de surface P30 et P22 purifiées.

Des auteurs ont montré que la P30 constitue l'antigène de surface majeur (voir Kasper et al., J.Immunol. 1983, *130,*2407-2412) et peut être utilisée pour la production de vaccins ou dans des tests de diagnostic notamment dans des immunoessais. Par ailleurs, Boothroyd et al. (voir la demande de brevet WO 89/08700) ont identifié et obtenu le matériel génétique codant pour la P30 de *Toxoplasma gondii* et suggèrent l'utilisation du gène pour la production de protéine recombinante, de peptides et d'anticorps. Ce gène a été cloné (Burg et al., 1988, J. Immunol., *141,* 3584-3591). L'analyse de la séquence montre un signal de sécrétion positionné à l'extrémité N-terminale qui est clivé dans la protéine P30 mature et une région C-terminale fortement hydrophobe également clivée et échangée par un glycolipide permettant son ancrage membranaire et un site potentiel de N-glycosylation.

Il subsiste cependant un problème qui consiste en l'obtention de quantités suffisantes de l'antigène P30 à la fois pour la préparation de vaccins et pour une utilisation dans des tests immunologiques. Kim et al. (Infection and Immunity, janvier 1994, 62, 203-209) ont décrit l'expression d'une P30 recombinante de conformation similaire à celle de la protéine native dans les cellules CHO sans toutefois réussir à obtenir des taux d'expression satisfaisants.

Il existe par ailleurs un intérêt à produire une P30 qui soit secrétée dans le milieu de culture pour faciliter son obtention et son utilisation pour la préparation de tests de diagnostic ou de compositions pharmaceutiques.

Par conséquent, la présente invention a pour objet une cassette d'expression fonctionnelle dans une cellule issue d'un organisme eucaryote non mammifère permettant l'expression d'un fragment d'ADN codant pour une protéine P30 de *Toxoplasma gondii,* placé sous le contrôle des éléments nécessaires à son expression ; ladite protéine P30 étant sécrétée de ladite cellule issue d'un organisme eucaryote et reconnue par des antisera humains.

D'une façon générale, toute cellule issue d'un organisme eucaryote non mammifère peut être utilisée dans le cadre de la présente invention et tout particulièrement une cellule d'insecte ou une cellule issue d'un organisme eucaryote inférieur. Le terme "cellule issue d'un organisme eucaryote inférieur" fait référence à une cellule issue d'un organisme eucaryote unicellulaire ou pluricellulaire ne possédant pas de mécanisme permettant la différentiation cellulaire. De telles cellules sont connues de l'homme de l'art. On préférera néanmoins avoir recours à un champignon, notamment unicellulaire, ou à une levure, notamment de la souche *Kluyveromyces, Pichia, Hasegawaea, Saccharomyces* ou *Schizosaccharomyces* et tout particulièrement sélectionnée parmi le groupe constitué de *Saccharomyces cerevisiae, Schizosaccharomyces pombe, Schizosaccharomyces malidevorans,* *Schizosaccharomyces sloofiae, Schizosaccharomyces octosporus* et *Hasegawaea japonicus*. Bien entendu, ces exemples ne sont pas limitatifs. Un grand nombre de ces cellules sont disponibles commercialement dans des collections telles que l'ATCC (Rockville, MA, USA) et l'AFRC (Agriculture and Food Research Council, Norfolk, UK).

Aux fins de la présente invention, ladite cellule peut être de type sauvage ou mutante. Dans ce contexte, on préfère particulièrement une cellule mutante auxotrophe ayant perdu la capacité à synthétiser au moins un métabolite essentiel à sa croissance, de sorte qu'elle ne peut croître que dans un milieu supplémenté par ce métabolite spécifique ou par complémentation avec un gène permettant sa synthèse. Bien que l'on connaisse à ce jour un très grand nombre de mutations d'auxotrophie pour différents métabolites essentiels, on peut citer plus particulièrement les mutations inhibant la synthèse de l'arginine, de la leucine et de l'uracile. De telles mutations sont décrites dans la littérature accessible à l'homme du métier. On peut généralement générer un grand nombre de mutations d'auxotrophie affectant diverses voies de biosynthèse en appliquant l'approche suivante. Brièvement, il suffit de cultiver les cellules de type sauvage traitées à l'aide d'un mutagène, parallèlement en présence et en absence du métabolite essentiel ciblé et de rechercher les mutants qui ne se multiplieront qu'en sa présence contrairement aux cellules sauvages qui peuvent croître dans les deux cas.

Une cassette d'expression selon l'invention, est destinée à la production d'une protéine P30 sous forme sécrétée de la cellule issue d'un organisme eucaryote non-mammifère et reconnue par des antisera anti-toxoplasmes. De tels antisera proviennent de patients ayant contracté une toxoplasmose récente ou éloignée et contenant des immunoglobulines reconnaissant les antigènes de *Toxoplasma gondii* et notamment la P30. Il va de soi que ladite protéine P30 peut également être reconnue par d'autres anticorps dirigés contre la protéine P30 naturelle, comme par exemple des anticorps monoclonaux ou polyclonaux obtenus par immunisation d'espèces variées avec la protéine naturelle.

Par protéine P30 on entend l'antigène de surface de *Toxoplasma gondii* produit par les techniques de recombinaison génétique décrites dans la présente demande ou tout fragment ou mutant de cet antigène à la condition qu'il soit immunologiquement réactif avec des anticorps dirigés contre la protéine P30 de ce parasite. De manière avantageuse, une telle protéine possède une séquence en acides aminés présentant un degré d'homologie d'au moins 70%, de préférence d'au moins 85% et, de manière tout à fait préférée, d'au moins 95% par rapport à la séquence spécifiée par Burg et al. (1988, *supra*). En pratique, un tel équivalent peut être obtenu par délétion, substitution et/ou addition de un ou plusieurs acide(s) aminé(s) de la protéine native. L'homme du métier connaît les techniques qui permettent d'effectuer ces modifications sans affecter la reconnaissance immunologique.

Un fragment d'ADN en usage aux fins de la présente invention, peut être obtenu par toute technique en usage dans le domaine de l'art, par exemple par clonage d'une banque d'ADN génomique de *Toxoplasma gondii* ou d'ADN complémentaire (ADNc) avec une sonde adéquate, par PCR (Polymerase Chain Réaction) ou encore par synthèse chimique.

Dans le cadre de la présente invention, on aura recours à une protéine P30 amputée de tout ou partie de la région C-terminale hydrophobe et comprenant les éléments appropriés pour permettre la sécrétion, comme par exemple un signal de sécrétion. Bien entendu, ce dernier peut être homologue c'est à dire issu de la protéine P30 native. Dans ce contexte, une cassette d'expression préférée selon l'invention permettra la production d'une protéine P30 ayant la séquence telle que montrée dans l'identificateur de séquence NO: 1 débutant à l'acide aminé +1 et se terminant à l'acide aminé +299. Comme mentionné précédemment, il peut également s'agir d'un mutant ou d'un fragment de ladite protéine P30.

De manière alternative, on peut également employer un signal de sécrétion hétérologue c'est à dire issu d'une protéine sécrétée ou membranaire quelconque, à la condition toutefois qu'il soit fonctionnel dans la cellule issue d'un organisme eucaryote non-mammifère considérée. Selon cette variante, une cassette d'expression selon l'invention comprend un fragment d'ADN codant pour une protéine P30 ayant la séquence telle que montrée dans l'identificateur de séquence NO: 1 débutant à l'acide aminé +31 et se terminant à l'acide aminé + 299 ou un fragment ou mutant de ladite protéine P30, ledit fragment d'ADN comprenant, en outre, une séquence codant pour un signal de secrétion hétérologue. Ce dernier est généralement placé en amont du premier résidu N-terminal de la protéine P30 mature, à savoir le résidu 31 de la SEQ ID NO: 1. Le choix d'un signal de secrétion est large et à la portée de l'homme de l'art.

A titre indicatif, on cite celui de la phosphatase acide majeure (pho1) de *Schizosaccharomyces pombe* (Elliot et al., 1986, J. Biol. Chem. *261,* 2936-2941) et la séquence pré-pro de la phéromone sexuelle alpha (Mating Factor alpha ou MFα) de *Saccharomyces cerevisiae* (Kurjan et Herskowitz, 1982, Cell, *30*, 933-934).

Selon un mode de réalisation avantageux, une cassette d'expression selon l'invention permet la production d'une protéine P30 de *Toxoplasma gondii* non-glycosylée. Bien que toutes les méthodes conventionnelles puissent être mises en oeuvre pour inhiber la N-glycosylation dans la cellule issue d'un organisme eucaryote non-mammifère considérée, comme par exemple, l'ajout de tunicamycine dans le milieu de culture, on préfère cependant muter le site potentiel de N-glycosylation afin qu'il ne soit plus reconnu par les enzymes cellulaires participant à la glycosylation. A cet effet, on met de préférence, en oeuvre un fragment d'ADN codant pour une protéine P30 comprenant au moins une mutation ; ladite mutation étant caractérisée par la présence d'un résidu acide aminé différent du résidu naturel en position 241 et/ou 243 de la séquence telle que montrée dans l'identificateur de séquence NO: 1, à la condition que le résidu acide aminé en position 243 ne soit pas une thréonine. Un mutant de la protéine P30 particulièrement préféré dans le cadre de la présente invention comprend un résidu glutamine en position 241, à la place du résidu asparagine naturel.

Bien entendu, une cassette d'expression selon l'invention peut permettre la production d'une protéine P30 (ayant une séquence en acides aminés telle que spécifiée précédemment) fusionnée à un élément exogène pouvant aider à sa stabilité, sa purification ou sa production. Le choix d'un tel élément est large et à la portée de l'homme du métier. Il peut notamment s'agir d'une protéine ou d'un peptide exogène. On peut citer, par exemple la protéine pho1 de *Schizosaccharomyces pombe,* la β-galactosidase, un enchainement de résidus lysine (poly Lys) ou histidine (poly His). La fusion peut avoir lieu en N ou en C-terminal de la protéine P30 en usage dans la présente invention.

Une cassette d'expression selon l'invention comprend des éléments nécessaires à l'expression dudit fragment d'ADN dans la cellule issue d'un organisme eucaryote non-mammifère considérée. Par "éléments nécessaires à l'expression", on entend l'ensemble des éléments qui permettent la transcription d'un fragment d'ADN en ARN messager (ARNm) et la traduction de ce dernier en protéine. Parmi ceux-ci, la région promotrice revêt une importance particulière. Elle peut être constitutive c'est à dire permettre un niveau de transcription constant tout au long du cycle cellulaire. A titre d'exemples non limitatifs, on indique les régions promotrices issues des gènes *PGK* (3-phosphoglycérate kinase) et *MFα* de *Saccharomyces cerevisiae* (Hitzeman et al., 1983, Science, *219*, 620-625), *adh* (alcool déshydrogénase) de *Schizosaccharomyces pombe* (Russel et Hall, 1983, J. Biol. Chem., 258, 143-149) et les promoteurs précoce p39K (Guarino et al., 1986, J. Virol., 57, 563) et tardif p12,5K (Hill-Perkins et al., 1990, J. Gen. Virol., *71*, 971-976) de baculovirus.

Cependant il peut être avantageux d'avoir recours à une région promotrice régulable permettant de faire varier les niveaux de transcription en fonction de conditions de culture ou de la phase de croissance cellulaire selon la présence d'un inducteur (activation de la transcription) ou d'un répresseur (répression). D'une manière générale, les régions promotrices régulables sont issues de gènes régulables pour lesquels les mécanismes de régulation peuvent être très variés. S'agissant de *Schizosaccharomyces pombe,* on peut citer les gènes de choc thermique dont l'expression augmente avec la température (Gallo et al., 1991, Mol. Cell. Biol., *11,* 281-288) et le gène codant pour l'enzyme fructose biphosphatase (*fbp*) dont la transcription est réprimée en présence de glucose et induite en condition de carence (Hoffman et Winston, 1989, Gene, *84,* 473-479). Entrent également dans cette catégorie, les gènes régulables par la thiamine comme les gènes *pho4* (Yang et Schweingruber, 1990, Curr. Genet., 18, 269-272), *nmt1* (Maundrell, 1990, J. Biol. Chem., *265,* 10857-10864) et *thi2* (Zurlinden et Schweingruber, 1992, Gene, *117,* 141-143) dont l'expression est régulée au niveau transcriptionnel par la thiamine, plus précisemment réprimée en présence de thiamine et induite ou déréprimée en son absence.

S'agissant d'une région promotrice régulable, on préfère tout particulièrement avoir recours à une région promotrice régulable par la thiamine. Cette dernière peut être isolée, par des techniques conventionnelles, de gènes répondant à ce type de régulation, tels que ceux mentionnés précédemment. Bien entendu elle peut être modifiée par mutation, délétion et/ou addition d'un ou plusieurs nucléotide(s) par rapport à la séquence de la région promotrice native, à la condition toutefois que ces modifications n'altèrent pas de façon drastique sa capacité de régulation. On peut également employer un fragment d'une telle région promotrice, notamment un fragment comportant les séquences d'activation et/ou de répression responsables de la régulation par la thiamine. Celui-ci est placé en amont d'une TATA box et d'un site d'initiation de la transcription conventionnels capables d'initier la transcription dans la cellule issue d'un organisme eucaryote non-mammifère considérée. Bien qu'un seul fragment de la région promotrice soit suffisant pour assurer la régulation par la thiamine, on peut également envisager, pour améliorer les niveaux d'expression, d'employer plusieurs fragments placés en tandem et dans une orientation quelconque par rapport à la TATA box.

Une cassette d'expression selon l'invention particulièrement intéressante est celle qui associe une région promotrice issue du gène *pho4* de *Schizosaccharomyces pombe* et un fragment d'ADN codant pour une protéine P30 telle que définie précédemment.

D'autre part, une cassette d'expression selon l'invention peut, en outre, contenir d'autres éléments contribuant à l'expression du fragment d'ADN, notamment une séquence de terminaison de la transcription telle que celle du gène *arg3* de *Schizosaccharomyces pombe* (Van Huffel et al., 1992, Eur. J. Biochem., *205,* 33-43) ainsi que des séquences activatrices de transcription.

Selon un mode de réalisation particulièrement avantageux, une cassette d'expression selon l'invention permet la production, sous forme sécrétée de la cellule non-mammifère, d'au moins 0,1 mg/l d'une protéine P30, avantageusement d'au moins 0,2 mg/l et de préférence d'au moins 0,3 mg/l.

La présente invention s'étend également à un vecteur comprenant une cassette d'expression selon l'invention. Il peut s'agit d'un vecteur viral et notamment d'un vecteur dérivé d'un baculovirus, tout particulièrement destiné à l'expression dans une cellule d'insecte.

Il peut également s'agir d'un vecteur plasmidique à replication autonome et en particulier d'un vecteur multicopies présent entre 5 et 500 copies dans la cellule hôte, avantageusement entre 10 et 400 copies et, de préférence, entre 20 et 300 copies. On cite, à titre d'exemple, les vecteurs dérivés de pGEM3 (Weilguny et al., 1991, Gene, 99, 47-54) et pFL20 (Losson et Lacroute, 1983, Cell, 32, 371-377). Par ailleurs un vecteur selon l'invention, peut également comprendre des éléments assurant sa réplication, tels que l'origine 2 de *Saccharomyces cerevisiae* ou *ars de Schizosaccharomyces pombe* et, de façon optionnelle, *ori* d'*Escherichia coli.* En outre, il peut également comprendre un gène de sélection, comme (i) un gène permettant la synthèse d'un métabolite essentiel, notamment le gène *URA3* ou *LEU2* de *Saccharomyces cerevisiae* et le gène *ura4* ou *leu1* de *Schizosaccharomyces pombe* ou (ii) un gène de résistance à un antibiotique.

La présente invention concerne également une cellule issue d'un organisme eucaryote non-mammifère comprenant une cassette d'expression selon l'invention soit sous forme intégrée dans le génome cellulaire ou insérée dans un vecteur. Une cellule selon l'invention a été définie précédemment. On préfère tout particulièrement, une cellule d'insecte, un champignon unicellulaire ou une levure, notamment une levure sélectionnée parmi le groupe constitué de *Saccharomyces cerevisiae, Schizosaccharomyces pombe, Schisosaccharomyces malidevorans, Schizosaccharomyces sloofiae, Schizosaccharomyces octosporus* et *Hasegawaea japonicus*.

La présente invention vise également une protéine P30 produite par une cassette d'expression, un vecteur ou une cellule issue d'un organisme eucaryote non-mammifère selon l'invention. Dans le cadre de la présente invention, la protéine P30 peut être modifiée *in vitro,* notamment par addition ou délétion de groupements chimiques, tels que des phosphates, sucres ou acide myristique de manière à améliorer sa stabilité ou la présentation d'un ou de plusieurs épitope(s).

La présente invention a également pour objet un procédé de préparation d'une protéine P30, selon lequel :
(i) on cultive dans des conditions appropriées une cellule issue d'un organisme eucaryote non-mammifère ; et
(ii) on récupère ladite protéine sécrétée de ladite cellule issue d'un organisme eucaryote non-mammifère.

Dans le cadre de l'invention, la protéine P30 est récupérée directement dans le milieu de culture selon les techniques de purification conventionnelles, comme par exemple la chromatographie échangeuse d'ions ou d'interactions hydrophobes, la filtration sur gel ou l'immunopurification.

S'agissant de la variante selon laquelle la protéine P30 est produite en mettant en oeuvre une cassette d'expression comprenant une région promotrice régulable par la thiamine et, en particulier issue du gène *pho4* de *Schizosaccharomyces pombe,* les cellules hébergeant une telle cassette sont cultivées dans un milieu supplémenté en thiamine lorsque la culture vise uniquement leur propagation. Dès lors qu'une culture est entreprise en vue de produire une protéine P30 de *Toxoplasma gondii,* les cellules sont transférées dans un milieu dépourvu de thiamine. Une telle variante est plus particulièrement destinée à être mise en oeuvre avec des cellules eucaryotes inférieurs. Ainsi, selon un procédé de l'invention :
(i) on cultive dans des conditions appropriées en absence de thiamine une cellule issue d'un organisme eucaryote inférieur ; et
(ii) on récupère ladite protéine sécrétée de ladite cellule issue d'un organisme eucaryote inférieur.

La présente invention concerne aussi un réactif pour la détection et/ou la surveillance d'une infection par *Toxoplasma gondii,* qui comprend à titre de substance réactive une protéine recombinante telle que définie précédemment.

Le réactif ci-dessus est ou sera fixé directement ou indirectement sur un support solide approprié. Le support solide peut-être, sans limitation, sous la forme d'un cône, d'un tube, d'un puits, de billes ou analogues.

Le terme "support solide" tel qu'utilisé ici inclut tous les matériaux sur lesquels peut être immobilisé un réactif pour une utilisation dans des tests diagnostiques. Des matériaux naturels, de synthèse, modifiés chimiquement ou non peuvent être utilisés comme support solide, notamment les polysaccharides tels que les matériaux de cellulose, par exemple du papier, des dérivés de cellulose tels que l'acétate de cellulose et la nitrocellulose ; des polymères tels que chlorure de vinyle, polyéthylène, polystyrènes, polyacrylate ou copolymères tels que polymère de chlorure de vinyle et de propylène, polymère de chlorure de vinyle et acétate de vinyle ; copolymères à base de styrène ; des fibres naturelles telles que le coton et des fibres synthétiques telles que le nylon.

De préférence, le support solide est un polymère de polystyrène ou un copolymère de butadiène-styrène. Avantageusement, le support est un polystyrène ou un copolymère à base de styrène comprenant entre environ 10 et 90% en poids de motifs styrène.

La fixation du réactif sur le support solide peut être réalisée de manière directe ou indirecte.

De manière directe, deux approches sont possibles : soit par adsorption du réactif sur le support solide, c'est à dire par des liaisons non covalentes (principalement de type hydrogène, Van der Walls ou ionique) soit par établissement de liaisons covalentes entre le réactif et le support.

De manière indirecte, on peut fixer préalablement (par adsorption ou covalence) sur le support solide un composé "anti-réactif" capable d'interagir avec le réactif de façon à immobiliser l'ensemble sur le support solide. A titre d'exemple, on peut citer un anticorps anti-P30, à la condition qu'il soit immunologiquement réactif avec une partie de la protéine différente de celle intervenant dans la réaction de reconnaissance des anticorps des séra ; un système ligand-récepteur, par exemple en greffant sur la protéine P30 une molécule telle qu'une vitamine et en immobilisant sur la phase solide le récepteur correspondant (par exemple le système biotine - streptavidine) .
Par manière indirecte , on entend également le greffage préalable ou l'insertion par recombinaison génétique d'une protéine ou d'un polypeptide à une extrémité de la protéine P30 et l'immobilisation de cette dernière sur le support solide par adsorption passive ou covalence de la protéine ou du polypeptide.

L'invention concerne encore un procédé de détection d'anticorps anti-toxoplasme dans un échantillon biologique, tel qu'un échantillon de sang, d'un individu ou d'un animal susceptible d'être ou d'avoir été infecté par *Toxoplasma gondii,* selon lequel on réalise au moins les étapes suivantes:
- on prépare un mélange comprenant :
   i) un réactif tel que défini ci-dessus qui est ou qui sera immobilisé sur un support solide,
   il) l'échantillon,
   iii) une anti-immunoglobuline marquée;
- on incube le mélange pendant un temps prédéterminé;
- on sépare la phase solide de la phase liquide; et
- on met en évidence la présence éventuelle d'anticorps anti-toxoplasme en mesurant le degré de marquage dans la phase solide.
   Dans un mode de réalisation de l'invention,
- on prépare un mélange comprenant :
   i) le réactif immobilisé sur le support solide, et
   ii) l'échantillon;
- on incube le mélange pendant un temps prédéterminé permettant la formation d'un complexe immun immobilisé sur le support solide;
- on ajoute une anti-immunoglobuline marquée dans des conditions d'incubation appropriées permettant sa réaction avec le complexe immun immobilisé;
- on sépare la phase solide de la phase liquide; et
- on met en évidence la présence éventuelle d'anticorps anti-toxoplasme en mesurant le degré de marquage dans la phase solide.

Dans un autre mode de réalisation de l'invention, le procédé de détection d'anticorps anti-toxoplasme dans un échantillon biologique, tel qu'un échantillon de sang, d'un individu ou d'un animal susceptible d'être ou d'avoir été infecté par *Toxoplasme gondii* comprend au moins les étapes suivantes :
- on prépare un mélange comprenant :
   i) une anti-immunoglobuline qui est ou qui sera fixée sur un support solide;
   ii) l'échantillon;
   iii) un réactif marqué, qui comprend à titre de substance réactive la protéine P30 précédemment citée;
- on incube le mélange pendant un temps prédéterminé;
- on sépare la phase liquide de la phase solide; et
- on met en évidence la présence éventuelles d'anticorps anti-toxoplasme en mesurant le degré de marquage dans la phase solide.

Avantageusement, on prépare un mélange comprenant l'anti-immunoglobuline fixée sur le support solide et l'échantillon,
- on incube le mélange pendant un temps prédéterminé permettant la formation d'un complexe immun immobilisé sur le support solide;
- on sépare la phase liquide de la phase solide;
- on ajoute le réactif marqué comprenant à titre de substance réactive la protéine P30 précédemment citée; et
- on met en évidence la présence éventuelles d'anticorps anti-toxoplasme en mesurant le degré de marquage dans la phase solide.

A titre d'exemple, on peut citer comme marqueur une enzyme telle que la peroxydase de raifort, la phosphatase alcaline; un isotope radioactif tel que ¹²⁵I, ³H, ⁵⁷Co, un marqueur luminescent ou analogue.

L'invention concerne également des anticorps monoclonaux ou polyclonaux obtenus par réaction immunologique d'un organisme humain ou animal à un agent immunogène constitué par la protéine P30 recombinante et leur utilisation à titre de substance réactive dans un réactif pour la détection et/ou la surveillance d'une infection par *Toxoplasma gondii* dans un échantillon biologique, tel qu'un prélèvement tissulaire; les anticorps étant au préalable marqués par tout marqueur approprié tel que défini précédemment.

Aussi, l'invention a pour objet un procédé de détection de la protéine P30 de *Toxoplasma gondii* dans un échantillon biologique, tel qu'un prélèvement tissulaire, d'un individu ou d'un animal susceptible d'être ou d'avoir été infecté par *Toxoplasma gondii,* selon lequel on met en contact l'échantillon et un réactif tels que définis ci-dessus dans des conditions appropriées permettant une éventuelle réaction immunologique, et on détecte la présence éventuelle d'un complexe immun formé avec le réactif précité en mesurant le degré de marquage dans l'échantillon biologique.

L'invention a aussi pour objet une composition immunothérapeutique active, notamment une préparation vaccinale, qui comprend à titre de principe actif, une protéine P30 recombinante, le principe actif étant éventuellement conjugué avec un support pharmaceutiquement acceptable, et éventuellement un excipient et/ou un adjuvant approprié.

La présente invention couvre également une composition pharmaceutique destinée au traitement ou à la prévention d'une infection par *Toxoplasma gondii* chez un homme ou un animal, comprenant une quantité thérapeutiquement efficace d'une cassette d'expression, d'un vecteur, d'une cellule issue d'un organisme eucaryote non-mammifère ou d'une protéine P30 selon l'invention ou préparée selon un procédé selon l'invention.

Les exemples ci-après permettront de mettre en évidence d'autres caractéristiques et avantages de la présente invention. Ces exemples sont illustrés par référence aux figures suivantes.

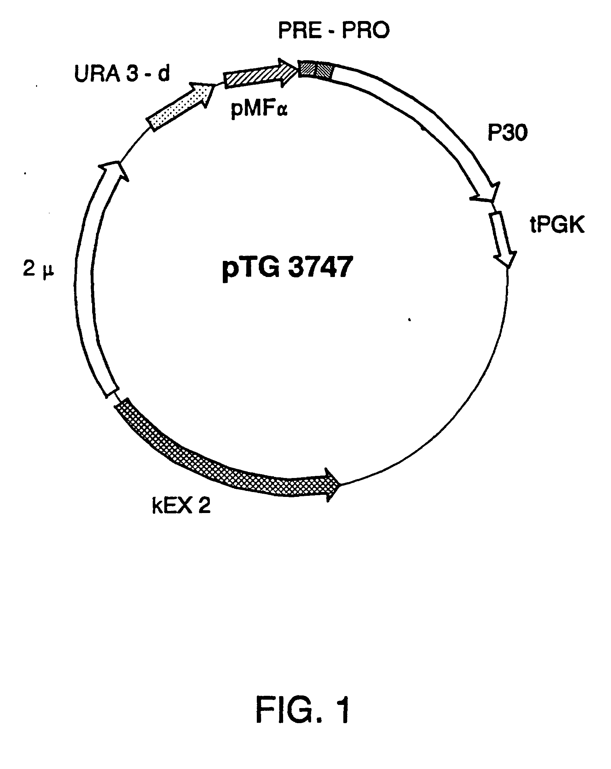

La Figure 1 est une représentation schématique du vecteur pTG3747 pour l'expression d'une protéine P30 de *Toxoplasma gondii* dans *Saccharomyces cerevisiae*.

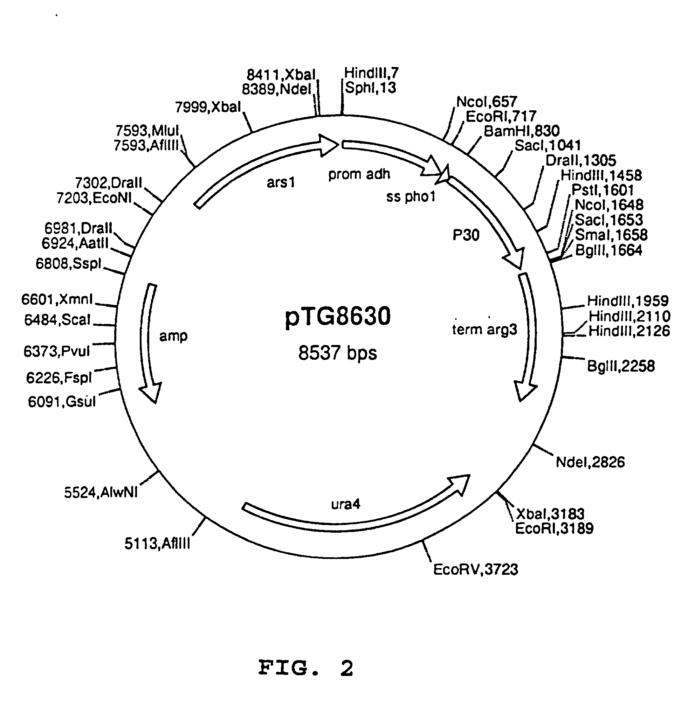

La Figure 2 est une représentation schématique du vecteur pTG8630 pour l'expression d'une protéine P30 non-glycosylée de *Toxoplasma gondii* dans une souche de *Schizosaccharomyces pombe* auxotrophe pour l'uracile.

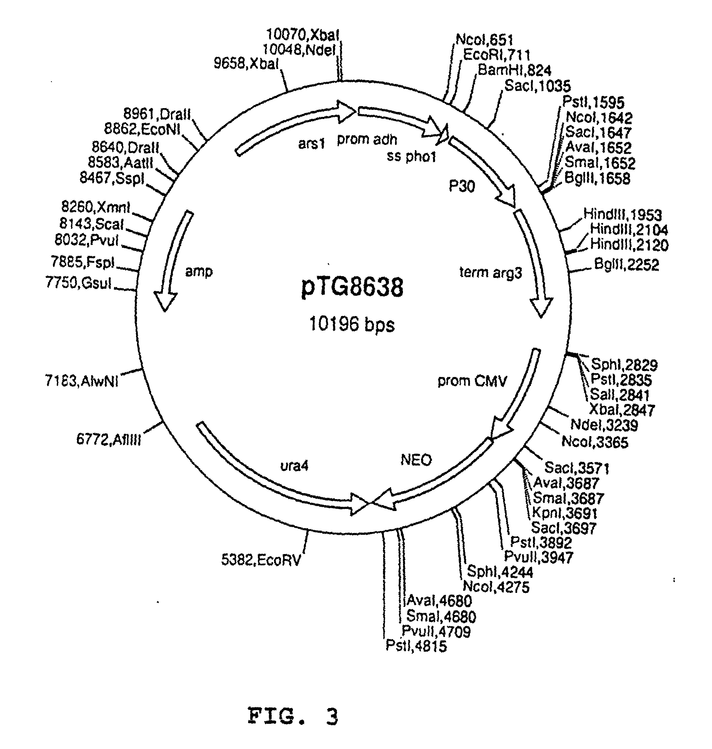

La Figure 3 est une représentation schématique du vecteur pTG8638 pour l'expression d'une protéine P30 de *Toxoplasma gondii* dans une souche de *Schizosaccharomyces pombe* de type sauvage.

### EXEMPLES :

Les constructions décrites ci-après ont été réalisées selon les techniques générales de génie génétique et de clonage moléculaire détaillées dans Maniatis et al. (1989, Laboratory Manual, Cold Spring Harbor Laboratory Press, Cold Spring Harbor, NY). L'ensemble des étapes de clonage mettant en oeuvre des plasmides bactériens est effectué par passage dans la souche *Escherichia coli (E. coli*) 5K. Quant aux étapes faisant intervenir un bactériophage de type M13, elles sont réalisées dans *E. coli* JM101.

On utilise la technique à l'acétate de lithium (Ito et al., 1983, J. Bacteriol., *153,* 163-168) pour introduire les différents vecteurs dans les souches de *Schizosaccharomyces pombe* et *Saccharomyces cerevisiae*. Mais, tout autre technique standard peut également être employée. D'autre part, en ce qui concerne le gène codant pour la protéine P30, la position des nucléotides correspond à celle spécifiée dans Burg et al. (1988, *supra*).

### EXEMPLE 1 : Production d'une protéine P30 dans Saccharomyces cerevisiae

### 1. Construction du vecteur d'expression pTG3747.

On isole les séquences codant pour la protéine P30 de *Toxoplasma gondii* d'un plasmide de l'art antérieur comprenant le gène correspondant (gène P30) s'étendant au moins des nucléotides 334 à 1632 . Ce plasmide de départ est désigné ci-après pbM 89.

On génère par PCR (Polymerase Chain Reaction) un fragment d'ADN codant pour une protéine P30 mature, dépourvue du peptide signal naturel et ayant un codon stop dans la partie C terminale juste en amont du nucléotide 1258. On utilise à titre de matrice le vecteur pbM89 et les amorces 1367 portant en 5' un site *Hin*dIII (SEQ ID NO: 2) et 1366 portant en 5' un site *Sal*I (SEQ ID NO: 3). Parallèlement, on isole du vecteur M13TG3868 un fragment *Sph*I-*Hin*dIII qui comporte le promoteur du gène *MFα* suivi des séquences codant pour le peptide pré-pro du même gène. Le vecteur M13TG3868 est issu du vecteur M13TG3841, décrit dans la publication de la demande internationale WO 90/13646, dans lequel on a introduit un site *Hin*dIII en 3' de la séquence pro *MFα*. Une telle modification est à la portée de l'homme de l'art. Ce fragment *Sph*I-*Hin*dIII ainsi que le produit d'amplification digéré par *Hin*dIII et *Sal*I sont clonés entre les sites *Sph*I et *Sal*I du vecteur pTG4812. Ce dernier dérive du vecteur pTG3828, décrit dans la demande européenne EPA 396 436, dans lequel on a introduit le gène *KEX2* codant pour une endo-protéase capable de cliver les séquences pré puis pro *MF*α (séquence indiquée dans ce même document européen). On obtient pTG3747 qui contient, notamment, le promoteur *MFα* dirigeant l'expression d'un fragment comportant les séquences pré-pro *MFα* suivies des séquences codant pour la P30 mature. Le gène de selection permettant l'isolement des clones est constitué par le gène *URA3* de *Saccharomyces cerevisiae*.
Bien entendu, il est à la portée de l'homme de l'art de recloner le gène de la P30, préalablement amplifié par PCR, à partir d'ADN extrait de Toxoplasma gondii dans des plasmides appropriés en vue de l'obtention de nouvelles constructions.

### 2. Production de la protéine P30 par Saccharomyces cerevisiae.

pTG3747 est introduit dans une souche de levure de l'espèce *Saccharomyces cerevisiae,* comme la souche TGY 73.4 de génotype *MFα, pra1, prb1, prc1,* *csp1, ura3, his3, pep4-3*. La souche non transformée se développe en milieu riche, par exemple le milieu YPG (1% de Yeast extract, 1% de bactopeptone DIFCO et 2% de glucose). Après transformation par le vecteur pTG3747, on sélectionne les prototrophes Ura + sur un milieu minimum, par exemple le milieu YNBG (contenant 0,7% de bases azotées pour levure (Yeast Nitrogen Base DIFCO), 0,5% de casamino acides et 1% de glucose). Les colonies sélectionnées sont analysées de la façon suivante. Après 24 h de pré-culture en milieu minimum, on dilue les cellules de manière à avoir une DO₆₀₀ₙₘ d'environ 1 et la culture est poursuivie dans les mêmes conditions pendant environ 72 h. On sépare cellules et surnageant par centrifugation.
Une aliquote de surnageant de culture est précipitée soit à l'acide trichloroacétique (0,3M final) dans le cas d'une reconnaissance avec un anticorps d'origine animale ou au PEG-4000 (20% final) dans le cas d'une reconnaissance avec un anticorps d'origine humaine. Le culot de précipitation est repris dans un tampon de dépôt (environ 1/200 du volume de départ) ne comprenant pas d'agent réducteur (-mercaptoéthanol ou DTT). L'analyse est effectuée par Western blot après une migration électrophorétique sur gel SDS-PAGE 12%, suivi d'un transfert sur une membrane de nitrocellulose (0,45 µm, Schleicher Schüll). Celle-ci est incubée avec un sérum polyclonal de lapin anti-toxoplasme positif, un antisérum humain antitoxo positif ou l'anticorps monoclonal 1E1E7 qui reconnaît un épitope de la protéine P30 (Fortier et al., 1991, Eur. J. Microbiol. Infect. Dis., *10,* 38-40). La réaction est révélée par un conjugué anti-lapin, anti-humain ou anti-souris marqué à la phosphatase alcaline (Immunoresearch).

Lorsqu'on analyse les surnageants de culture de la souche TGY 73.4, on détecte une protéine P30 recombinante se présentant sous forme d'un matériel hétérogène, probablement du fait de clivages protéolytiques par l'enzyme KEX2 ou d'une grande hétérogénéité dans la glycosylation, mais néanmoins reconnue en Western blot par des antisera humains anti-toxoplasmes positifs.

### EXEMPLE 2 : Production d'une protéine P30 chez Schizosaccharomyces pombe

### 1. Construction du vecteur d'expression pTG8610 (expression constitutive dans une souche auxotrophe)

Le plasmide pEVp11 (Russell et Nurse, 1986, Cell, *45,* 145-153) contient la région promotrice du gène *adh* de *Schizosaccharomyces pombe* sous forme d'un fragment *SphI-Eco*RI de 700pb, le site *Eco*RI étant situé dans la région 5' du gène *adh,* 59pb en amont de l'ATG initiateur. Il est digéré par les enzymes *Eco*RI et *Hin*dIII et on introduit un fragment synthétique résultant de la réassociation des oligonucléotides simple brin OTG2781 et OTG2782 (décrits respectivement dans les SEQ ID NO: 4 et NO: 5), dans le but de compléter la région promotrice *adh* jusqu'à 11 pb en amont de l'ATG initiateur et de créer des sites de restriction appropriés facilitant les étapes de clonage ultérieures. On génère pTG1702.

Un fragment *Hpa*I*-Cla*I de 0,92 kb est isolé de pCVH3 (Van Huffel et al., 1992, Eur J. Biochem., *205,* 33-43) puis traité au fragment Klenow de l'ADN polymérase. Ce fragment comporte les derniers codons du gène *arg3* de *Schizosaccharomyces pombe* suivis de la séquence de terminaison de la transcription. Il est inséré dans pTG1702 digéré par *Hin*dIII et dont les extrémités ont été rendues franches par traitement au fragment Klenow de l'ADN polymérase, pour donner pTG1746.

pTG1746 est digéré par *Xba*I, traité au fragment Klenow de l'ADN polymérase avant d'être digéré par *Bam*HI. Le fragment comportant les séquences de terminaison de la transcription est introduit dans le vecteur pDW230 digéré par *Eco*RI, soumis à un traitement au fragment Klenow de l'ADN polymérase puis digéré par *Bam*HI. On obtient pTG1751.

Le vecteur pDW230 est similaire à pDW232 décrit dans la littérature (Weilguny et al., 1991, Gene, *99,* 47-54). Ils dérivent tous deux de pGEM3 dans lequel on a inséré une origine de réplication fonctionnelle dans *Schizosaccharomyces pombe* (origine *ars1*) ainsi que le gène *ura4* de *Schizosaccharomyces pombe* en tant que marqueur de sélection ; à la différence que l'introduction du fragment portant l'origine *ars1* au niveau du site *Nae*I de pGEM3, donnant lieu à pDW230, a entraîné une délétion de ce site jusqu'à la base 2862.

On génère le vecteur pTG1754 par ligation des fragments *Sph*I *- Bam*HI de pTG1751 (comportant la séquence de terminaison de la transcription *arg*3) et de pTG1702 (comprenant le promoteur *adh*).

Parallèlement, les séquences codant pour la P30 munie de son propre signal de sécrétion sont obtenues de la façon suivante : on modifie le gène P30 par mutagénèse, de manière à générer une séquence consensus d'initiation pour la levure au niveau du second ATG initiateur (TAAAAAATGTCT) et un codon stop à la même position que précédemment. La PCR met en oeuvre le vecteur pbM89 en tant que matrice et les oligonucléotides 722 (SEQ ID NO: 6) et 723 (SEQ ID NO: 7) portant à leur extrémité 5' un site *Bgl*II. Le produit d'amplification traité par *Bgl*II, est cloné dans le vecteur pTG2886 linéarisé par l'enzyme *Bgl*II. Ce dernier est décrit dans la publication européenne EPA 396 436.1. Les clones recombinants sont analysés par digestion enzymatique *Hin*dIII-*Bam*HI afin de déterminer l'orientation de l'insert par rapport au promoteur. Un clone présentant l'orientation adéquate est désigné pTG2886-P30. Après digestion par *Bgl*II, le fragment comportant les séquences codant pour la protéine P30 munie de son propre signal de sécrétion, est inséré dans le vecteur pTG1754 préalablement digéré par *Bam*HI. On obtient pTG 3733.

On remplace la séquence codant pour le signal de sécrétion naturel de la protéine P30 (résidus +1 à +30 de la SEQ ID NO: 1) par la séquence du gène *pho1* de *Schizosaccharomyces pombe* codant pour le signal de sécrétion de la phosphatase. Pour ce faire, le vecteur pTG2886-P30 est digéré par *Bam*HI et on introduit un fragment synthétique présentant les mêmes extrémités protubérentes portant un site *Bgl*II interne en 5' et résultant de la réassociation des oligonucléotides OTG4096 et OTG4097 (SEQ ID NO: 8 et 9).

Le fragment *Bgl*II portant la séquence codant pour le signal de sécrétion pho1 suivi de la P30 mature (résidus + 31 à + 299 de la SEQ ID NO: 1) est isolé du vecteur obtenu dans l'étape précédente. Il est inséré dans le site *Bam*HI du vecteur pTG1754. Les transformants présentant l'orientation correcte vis à vis du promoteur *adh,* sont sélectionnés par digestion *Eco*RI-*Bam*HI. On génère pTG8610.

### 2. Construction du vecteur d'expression pTG8630 codant pour une protéine P30 non glycosylée (expression constitutive dans une souche auxotrophe)

Le fragment *Bam*HI*-Sma*I, comportant les séquences P30, est isolé de pTG8610 et introduit entre les mêmes sites de M13TG130 (Kieny et al., 1983, Gene, *26,* 91-99) afin de muter le site de glycosylation. On génère M13TG8620. La mutagenèse est effectuée à l'aide d'un kit commercial selon les recommandations du fournisseur (par exemple Amersham) et en mettant en oeuvre l'oligonucléotide OTG5829 (SEQ ID NO: 10). La mutagenèse a pour but de substituer le résidu asparagine en position 241 par une glutamine tout en créant un site *Hin*dIII, facilitant la sélection des mutants. On obtient M13TG8622.

On constitue le vecteur d'expression pTG8630 par insertion entre les sites *Sph*I*-Pst*I du vecteur pTG8610 d'un premier fragment *SphI-Dra*II obtenu de pTG8610 et d'un deuxième fragment *Dra*II*-Pst*I purifié de M13TG8622.

### 3. Construction du vecteur d'expression pTG8644 (expression régulable par la thiamine dans une souche auxotrophe)

On insère entre les sites *Bam*HI et *Sac*I du vecteur pTG1702 (exemple 2.1) un fragment d'ADN synthétique issu de la réassociation des oligonucléotides OTG2872 et OTG2873 (SEQ ID NO: 11 et NO: 12). On génère pTG1716 comprenant la séquence codant pour le signal de sécrétion de la phosphatase acide majeure de *Schizosaccharomyces pombe* (pho1) en aval de la région promotrice *adh*.

pTG1716 est modifié par l'insertion entre les sites *Mlu*I et *Hin*dIII d'une séquence d'ADN codant pour le variant Lys47 de l'hirudine (HV2 Lys47). On obtient pTG1722.

Le fragment *Sph*I*-Sac*I comportant la région promotrice *adh* suivie de la séquence codant pour le signal de sécrétion pho1 et HV2 Lys 47 est isolé de pTG1722. Il est ensuite sous-cloné entre les mêmes sites de pTG1751. Il en résulte pTG1757.

D'autre part, la région promotrice du gène *pho*4 de *Schizosaccharomyces pombe* est obtenue par PCR à partir du clone pSp4B (Yang and Schweingruber, 1990, Current Genet., 18, 269-272) et à l'aide des amorces OTG3569 comportant un site *Sph*I (SEQ ID NO: 13) et OTG3239 muni d'un site *Bam*HI (SEQ ID NO: 14). Le fragment *Sph*I*-Bam*HI ainsi généré est substitué au fragment *Sph*I*-Bam*HI portant la région promotrice *adh* de pTG1757 pour donner pTG2734.

On isole par PCR et à partir du vecteur pTG2734, un fragment de 642 pb comportant les séquences 5' flanquantes du gène *pho*4 de *Schizosaccharomyces pombe* situées en amont de la TATA box. On met en oeuvre les amorces OTG3569 (SEQ ID NO: 13) et OTG3210 (SEQ ID NO: 15). Le fragment PCR *Sph*I*-Nco*I ainsi obtenu, est introduit dans pTG1757 digéré par les mêmes enzymes pour donner pTG2735.

Enfin, on introduit le fragment *EcoRI* isolé de pTG8610 et comportant la séquence codant pour la protéine P30 suivie du terminateur *arg3* dans le vecteur pTG2735 digéré par *EcoRI.* On obtient le vecteur pTG8644 dans lequel les séquences codant pour le signal de sécrétion pho1 et la protéine P30 (résidus +31 à +299 de la SEQ ID NO: 1) sont sous le contrôle d'un promoteur hybride constitué par les séquences du gène *pho4* responsables de la régulation par la thiamine placées en amont de la TATA box du gène *adh*.

### 4. Construction du vecteur d'expression pTG8638 (expression constitutive dans une souche sauvage).

Il s'agit de générer des vecteurs d'expression tels que ceux des exemples 2.1, 2.2 ou 2.3 comportant, en outre, un gène permettant la sélection dans une souche sauvage ne présentant pas d'auxotrophie, par exemple un gène de résistance à un antibiotique, comme le gène *neo* (Néomycine) conférant la résistance au G418.

Le gène *neo* est issu du vecteur Tn5 (Berck et al., 1982, Gene, *19*, 327-336). Celui-ci est modifié par les techniques de mutagénèse classiques afin de créer des sites de restriction facilitant les étapes de clonage ultérieures, comme un site *Bam*HI aux extrémités 5' (en position 138) et 3' (en position 1280) du gène *neo.* Le fragment *Bam*HI est traité par l'ADN polymérase Klenow avant d'être introduit dans le site *Eco*RI de pDW230 dont les extrémités ont été rendues franches par traitement à la Klenow. On obtient pTG1796.

Le gène *neo* est placé sous le contrôle du promoteur IE1 de CMV (cytomegalovirus) s'étendant des nucléotides -727 à +78 (Boshart et al., 1985, Cell, 41, 521-530). On introduit en 5' du promoteur un site *Bam*HI et en 3' un site *Hin*dIII. Le fragment *Bam*HI-*Hin*dIII traité par l'ADN polymérase Klenow, est inséré en amont du gène *neo* dans le site *Bam*HI rendu franc par traitement par le Klenow de pTG1796. On génère pTG3777 dans lequel on pourra introduire les cassettes d'expression de la protéine P30 décrites précédemment.

Le fragment *Sph*I*-Nde*I comportant la cassette d'expression "promoteur *adh*-P30-term arg3" est isolé de pTG8610 puis traité à l'ADN polymérase T4. Il est cloné dans le site *Hin*dIII traité à l'ADN polymérase T4, de pTG3777 pour donner le vecteur pTG8638.

### 5. Production de la protéine P30 dans une souche de Schizosaccharomyces pombe auxotrophe.

Les vecteurs d'expression des exemples 2.1, 2.2 et 2.3 sont introduits dans une souche de *Schizosaccharomyces pombe* mutante, par exemple la souche D18 auxotrophe pour l'uracile et disponible à l'AFRC sous la référence 2036. On utilise, à titre de contrôle négatif, la même souche transformée en parallèle avec le vecteur pTG1754. Bien entendu, tout autre souche présentant ce type d'auxotrophie pourrait convenir.

Les souches pour lesquelles l'expression de la P30 est constitutive, à savoir celles transformées par les vecteur pTG8610 et pTG8630, sont cultivées à 30°C dans un milieu synthétique Kappeli optimisé pour la levure (Fiechter et al., 1981, Adv., Microbiol. Physiol. *22*, 123-183) et supplémenté avec 2% du glucose et un mélange de vitamines.

Les souches transformées par le vecteur pTG8644 sont pré-cultivées dans le même milieu Kappeli supplémenté. Cependant, le mélange de vitamines comprend notamment de la thiamine à une concentration finale de 0,002g/l (milieu thi⁺). Lorsque les cultures atteignent une DO (densité optique) à 600 nm comprise entre 1 et 2 , elles sont diluées à une DO d'environ 0,05 soit dans du milieu thi⁺ soit dans un milieu Kappeli supplémenté avec 2% de glucose et un mélange de vitamines dépourvu en thiamine (milieu thi-). La culture est poursuivie à 30°C jusqu'en fin de phase exponentielle.

Des aliquotes de chacune des cultures sont prélevées régulièrement durant la phase exponentielle ainsi qu'en fin de croissance. Les surnageants de culture sont analysés par Western blot, comme décrit dans l'exemple 1.2.

Les levures transformées par pTG8610 ou pTG3733 synthétisent et sécrètent une P30 recombinante reconnue indifféremment par les trois anticorps cités précédemment (antisérum de lapin, antisérum humain toxo positifs et anticorps 1E1E7). La P30 recombinante se présente sous forme d'un matériel homogène d'aspect légèrement diffus d'une masse moléculaire apparente (MMA) d'environ 35kDa. Un signal plus intense est observé dans le surnageant de culture de pTG8610 indiquant que l'efficacité du signal de sécrétion pho1 est supérieur pour la sécrétion de la protéine P30 dans *Schizosaccharomyces pombe* à celle du signal naturel de la P30. On peut noter que, dans des conditions réductrices (le tampon de dépôt contient un agent réducteur), les anticorps ne reconnaissent plus la P30 recombinante, indiquant que seuls des épitopes conformationels sont impliqués dans la reconnaissance en Western blot, comme cela est observé avec la P30 native.

De même, on détecte une production de protéine P30 recombinante dans les surnageants de culture de *Schizosaccharomyces pombe* transformée par pTG8644, ceci lorsque la culture est effectuée en absence de thiamine. Elle est révélée par les antiséra humains et présente une MMA de 35 kDa. Par contre, aucun produit n'est visualisé par ces mêmes anticorps spécifiques de la protéine P30, lorsque la culture est effectuée en présence de thiamine.

L'analyse des surnageants de levures transformées par pTG8630, réalisé en utilisant un antisérum de lapin toxopositif, révèle une bande majoritaire d'environ 28kDa en conditions non réductrices, soit inférieure d'environ 5kDa à celle observée pour le produit d'expression de pTG8610 ou pTG8644. Cette différence de masse moléculaire s'explique par le fait qu'il s'agit d'une protéine non glycosylable puisque mutée au niveau du site de N-glycosylation.

### 6. Production de la protéine P30 dans une souche de Schizosaccharomyces pombe de type sauvage.

Différentes souches sauvages de *Schizosaccharomyces pombe* sont disponibles à l'AFRC. On cite à titre indicatif les souches 20 286 et 26 760 dans lesquelles on transforme le vecteur pTG8638. On sélectionne les transformants du fait de leur résistance au G418 (concentration de 0,2 à 1 mg/ml). Après culture en milieu liquide sélectif (milieu YPG contenant de la néomycine), on analyse les surnageants de culture par Western blot à l'aide des antiséra humains. Une bande majeure est détectée à la position attendue pour la P30 recombinante glycosylée.

### 7. Construction de pTG9643 (production réglable par la thiamine d'une protéine P30 non glycosylée).

Le fragment *Eco*R1 isolé de pTG8630 (exemple 2.2) et comportant la séquence codant pour la P30 non glycosylée suivie du terminateur *arg*3 a été introduit dans le vecteur pTG2735 (exemple 2.3) digéré par *Eco*R1. On génère pTG9643. La protéine de fusion peut être produite selon la même technologie que celle employée à l'exemple 2.5.

### 8. Construction de pTG8667 (production régulable par la thiamine d'une protéine P30 glycosylée fusionnée à une queue poly His en C-terminal).

Le vecteur pTG8644 (exemple 2.3 est digéré par *Pst*I et *Sma*I et on introduit un fragment synthétique issu de la réassociation des oligonucléotides 0TG6438 et 6439 (SEQ ID NO: 16 et 17). On génère pTG8667 qui comprend le promoteur hybride pho4/adh, les séquences codant pour le signal de sécrétion pho1 et la protéine P30 suivie de 6 résidus histidine formant une queue poly His. La protéine de fusion peut être produite selon la même technologie que celle employée à l'exemple 2.5.

### 9. Construction de pTG9618 (production régulable par la thiamine d'une protéine P30 non glycosylée fusionnée à une queue poly His en C-terminal).

Le fragment *Bam*H1*-Pst*1 du pTG8667 (contenant la séquence de la P30 glycosylée) est remplacé par un fragment équivalent isolé de pTG8630 (P30 non glycosylée). On génère ainsi pTG9618. La protéine de fusion peut être produite selon la même technologie que celle employée à l'exemple 2.5.

### 10. Construction de pTG8221 (production régulable par la thiamine d'une protéine P30 non glycosylée fusionnée à une queue poly His en N-terminal).

Le plasmide pTG9643 est digéré par *Bam*H1 et un fragment d'ADN synthétique provenant de la réassociation des oligonucléotides 0TG10055 et 10094 (SEQ ID NO: 18 et 19) est cloné au niveau de ce site. On obtient pTG8221 dans lequel 8 résidus histidine précédant la protéine P30.

### EXEMPLE 3 : Production d'une protéine P30 dans baculovirus

Le fragment d'ADN codant pour la protéine P30 munie de son peptide signal, est isolé de pTG2886-P30 après digestion par *Bgl*II et inséré dans un vecteur baculovirus ; par exemple le vecteur pACMP1 digéré par *Bam*HI (Hill-Perkins et Possee, 1990, J. Gen. Virol., *71,* 971-976) ou le vecteur décrit par Guaniro et al. (1986, J. Virol., 57, 563), pour donner respectivement pTG3729 et pTG3731

Les recombinants sont générés comme décrit par J.M VLAK et al. dans Proceedings of the Baculovirus and Recombinant Protein Production Workshop, "Baculovirus and recombinant protein production processes", March 29-April 1, Interlaken, Switzerland.

Les surnageants sont centrifugés 10 min à 1000 tpm (tours par minute) et stockés à -20°C avant d'être analysés en SDS-PAGE 12 % suivi d'un Western Blot et en radioimmunoprécipitation.

Pour cette dernière technique, la culture cellulaire est marquée avec 300µCi de tran ³⁵S-label (nom commercial : ICN Biomédical France réf. 51006) à 1 heure 30 ou 26 heures après l'infection . On récolte le surnageant 48 heures après l'infection. L'immunoprécipitation est réalisée suivant le protocole suivant. Les surnageants de culture sont centrifugés à 2000 tpm pendant 20 min, dilués au demi dans du tampon NET 2x (NP40 0,1 %, EDTA 1mM, Tris-HCl 50 mM pH8,0, NaCl 150 mM, Gelatine 0,25 %, NaN, 0,02 %, Aprotinine 2 %, PMSF 2mM) puis immunoprécipités avec soit des séra humains toxopositifs ou avec l'anticorps monoclonal 1E1E7 pendant 2 heures à 4°C, et adjonction finale de protéine A Sepharose (Pharmacia). Le précipité est lavé successivement avec les tampons NET1x, Tris-NP40, PBS 1x (Na₂HPO₄ 12H₂O 8mM KH₂PO₄ 2mM, NaCl 150 mM) et PBS 0,1x. L'analyse est effectuée après migration électrophorétique sur gel SDS-PAGE 12 % et autoradiographie. La P30 recombinante est observée dans le surnageant de culture des cellules infectées par les baculovirus recombinants.

Lorsqu'on analyse les surnageants de culture par Western blot en conditions non-réductrices, tant les séra humains toxopositifs que l'antisérum de lapin, reconnaissent un doublet composé d'une bande majeure d'une MMA d'environ 28kDa et d'une bande mineure d'une MMA d'environ 29kDa. Aucune différence significative n'est observée entre les protéines P30 recombinantes produites par les virus recombinants.
Par contre, aucun produit n'est détecté en conditions réduites par le DTT.

### EXEMPLE 4 : Purification de la protéine P30 produite chez Schizosaccharomyces pombe

### 1) Purification par chromatographie d'échange d'ions (technique en colonne).

Les surnageants de culture (volume 10 litres) obtenus comme décrit dans l'exemple 2 sont réduits de 20 à 50 fois par concentration sur fibres creuses ou sur cassettes d'ultrafiltration tangentielles (volume final : 500 à 200 ml) (seuil de coupure 10.000 daltons), et dialysés par cette même opération dans un tampon Tris, 50mM, pH 8,5 environ sans NaCl ou un tampon équivalent, qui sera utilisé dans l'étape suivante.

La deuxième étape consiste en une chromatographie d'échanges d'ions classique, par exemple sur une colonne de 100ml de gel DEAE TRISACRYL (DEAE: diéthylaminoéthyle) ou Hyper D, (commercialisée par la société Biosepra) ou DEAETSK (Merck) permettant de récupérer, par un gradient de force ionique de 0 à 1M de NaCl, les éluats qui sont testés en gel SDS PAGE et analysés en Western Blot. L'analyse permet de mettre en évidence la bande d'environ 30kD de la P30 recombinante.

Pour parfaire l'isolement de cette protéine recombinante une étape de chromatographie d'affinité peut être effectuée sur un support chromatographique couplé par covalence à l'anticorps monoclonal 1E1E7anti P30 . Après élution spécifique par variation du pH (pH 2,5 ou pH 11,5) le pic de protéine P30 obtenu est de pureté suffisante pour l'emploi de cette protéine dans des kits de diagnostic.

Lorsque la protéine P30 est fusionnée à une queue polyhistidine aussi bien en N qu'en C-terminal, on peut inclure une étape de chromatographie chélate de métal. Les supports adaptés à cette technologie sont connus de l'homme de l'art et sont disponibles commercialement. On peut citer à titre d'exemples la chélatine Sepharose (Pharmacia) à charger en ions métalliques et le chélate de Nickel (NINTA Agarose, Quiagen).

### EXEMPLE 5 : Réactivité vis à vis des anticorps.

La réactivité de la P30 recombinante a été testée contre 151 séra connus dont 95 étaient donnés comme séra positifs et 56 comme séra négatifs, en utilisant l'appareil VIDAS (Marque enregistrée), commercialisé par la Société bioMérieux SA.

### 1) Test de référence : VIDAS TOXO IgG

Le test automatisé sur le système Vidas permet la mesure quantitative des IgG anti-toxoplasmiques dans un sérum humain. Le principe du dosage associe la méthode immunoenzymatique à une détection finale en fluorescence (ELFA).
Toutes les étapes des réactions sont gérées par l'instrument. Sur un cône en K resin (copolymère butadiène-styrène), à usage unique qui sert à la fois de phase solide et de système de pipettage, est adsorbée une préparation comprenant les antigènes membranaire et cytoplasmique de *Toxoplasma gondii;* cette préparation étant obtenue par sonication de toxoplasmes complets. Les autres réactifs nécessaires au test de diagnostic sont pré-répartis dans une cartouche associée au cône. La phase d'incubation du sérum humain permet la fixation des IgG anti-toxoplasme si elles sont présentes ; puis une globuline anti IgG humaine (Boehringer ref: 1272 896) marquée à la phosphatase alcaline (Boehringer ref: 556 602) est ajoutée pour révéler la présence de ces éventuelles IgG anti-toxoplasme.
Le résultat ou titre est exprimé en UI/ml (Unités Internationales) par rapport à une courbe de calibration mémorisée dans le Vidas. Les résultats sont présentés dans le tableau qui suit (voir colonne Vidas-Titres).

### 2) Test en microplaque de la protéine P30 recombinante

La P30 (5ng/puits) à tester est fixée dans les puits d'une plaque de microtitration (Polylabo, ref: 13159). Les mêmes séra humains qui ont été testés sur l'appareil VIDAS comme décrit ci-dessus ont été incubés pendant 1 heure à 37° C, à la suite de quoi une globuline anti IgG humaine marquée à la peroxydase (réf. 815462) a été ajoutée pour révéler les éventuelles IgG sériques réagissant contre cette P30.
Les résultats sont donnés en densité optique lue à 492 nm et présentés dans le tableau qui suit (voir colonne P30 REC).

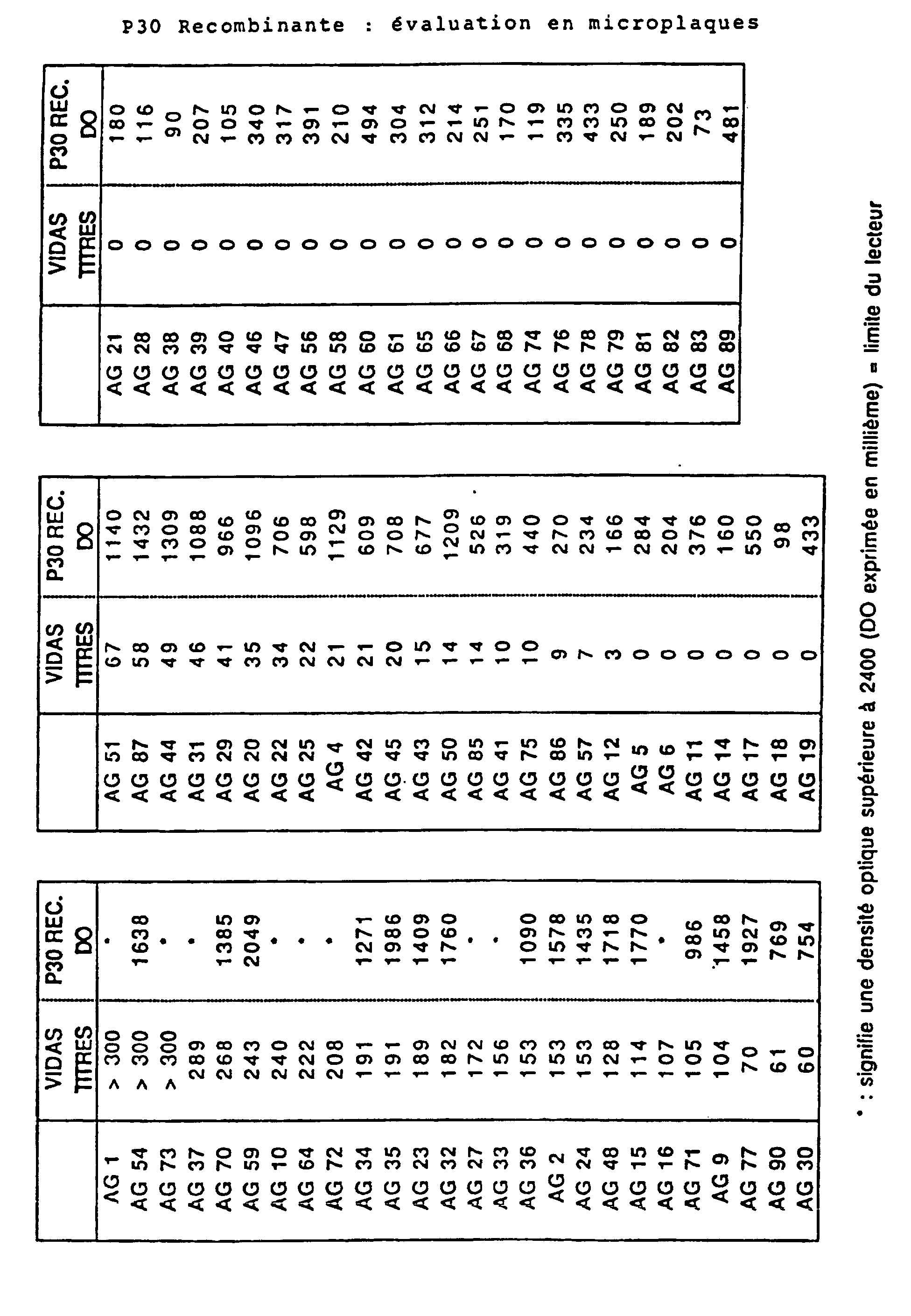

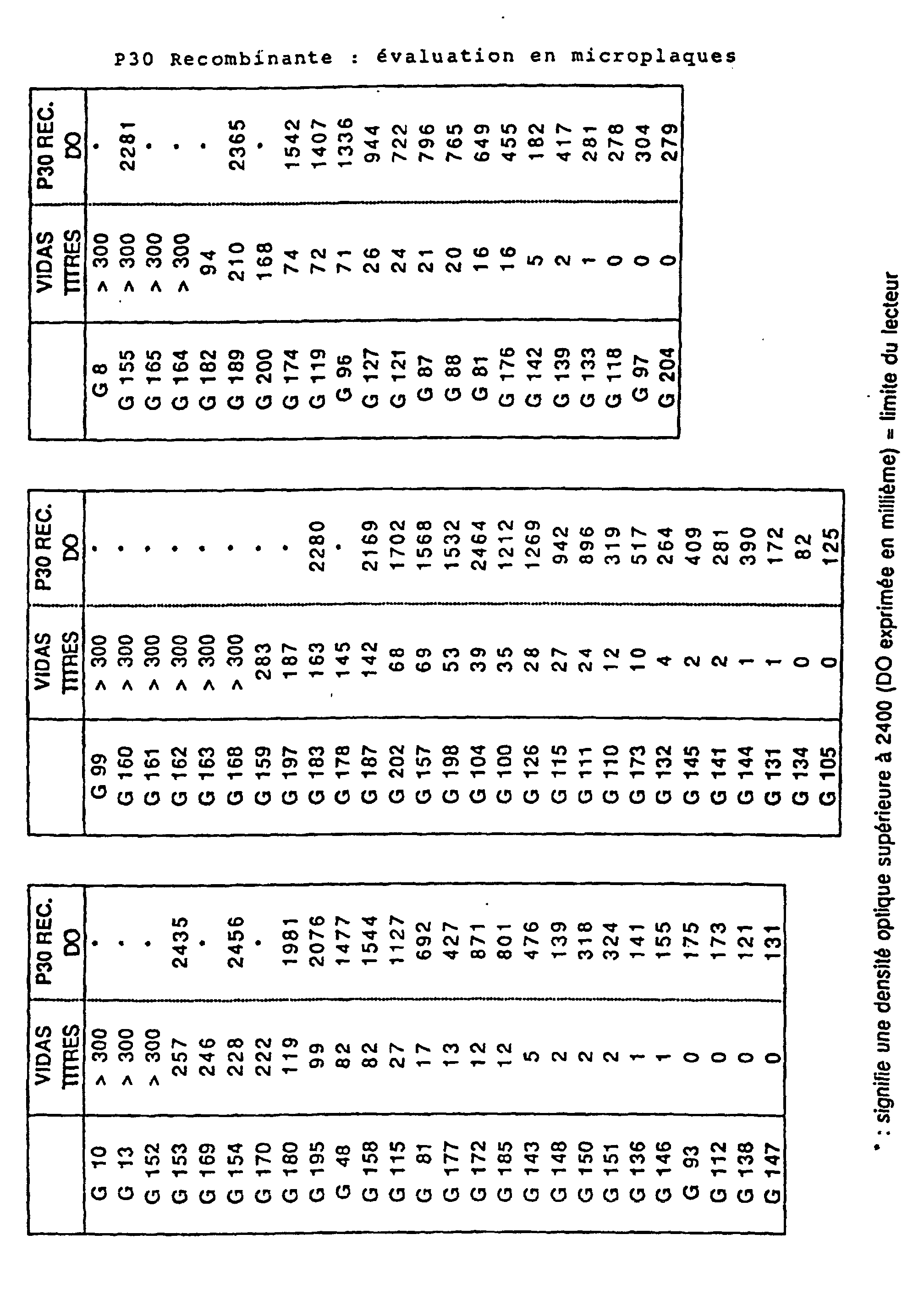

Comme on peut le voir à la lecture du tableau qui précède il existe une très bonne corrélation entre les résultats obtenus avec les tests effectués sur l'appareil VIDAS en présence d'antigènes membranaires et cytoplasmiques de Toxoplasme et ceux obtenus dans un essai classique sur microplaque en présence de la protéine P30 recombinante de l'invention.

## Revendications

1. Une cassette d'expression fonctionnelle dans une cellule issue d'un organisme eucaryote non-mammifère fragment d'ADN codant pour une protéine P30 de *Toxoplasma gondii,* placé sous le contrôle des éléments nécessaires à son expression ; ladite protéine P30 étant sécrétée de ladite cellule issue d'un organisme eucaryote et reconnue par des antiséra anti-toxoplasmes, **caractérisée en ce que** ledit fragment d'ADN code pour une protéine P30 amputée de toute la région C-terminale hydrophobe.

2. Une cassette d'expression selon la revendication 1, **caractérisée en ce qu'**elle est fonctionnelle dans une cellule d'insecte.

3. Une cassette d'expression selon la revendication 1, **caractérisée en ce qu'**elle est fonctionnelle dans une cellule issue d'un organisme eucaryote inférieur.

4. Une cassette d'expression selon la revendication 3, **caractérisée en ce que** la cellule issue d'un organisme eucaryote inférieur est une levure ou un champignon.

5. Une cassette d'expression selon la revendication 4, **caractérisée en ce que** la cellule issue d'un organisme eucaryote inférieur est sélectionnée parmi le groupe constitué de *Saccharomyces cerevisiae, Schizosaccharomyces pombe, Schizosaccharomyces malidevorans, Schizosaccharomyces sloofiae, Schizosaccharomyces octosporus* et *Hasegawaea japonicus*.

6. Une cassette d'expression selon l'une des revendications 1 à 5, **caractérisée en ce que** le fragment d'ADN code pour une protéine P30 ayant la séquence telle que montrée dans l'identificateur de séquence NO: 1 débutant à l'acide aminé +1 et se terminant à l'acide aminé +299 ou un équivalent immunologique de ladite protéine P30.

7. Une cassette d'expression selon l'une des revendications 1 à 5, **caractérisée en ce que** le fragment d'ADN code pour une protéine P30 ayant la séquence telle que montrée dans l'identificateur de séquence NO :1 débutant à l'acide aminé +31 et se terminant à l'acide aminé +299 ou un équivalent immunologique de ladite protéine P30, ledit fragment d'ADN comprenant, en outre, une séquence codant pour un signal de secrétion hétérologue.

8. Une cassette d'expression selon la revendication 7, **caractérisée en ce que** ladite séquence codant pour un signal de sécrétion hétérologue est issue du gène *pho1* de *Schizosaccharomyces pombe* ou du gène de la phéromone sexuelle alpha (Mating Factor *α, MFα*) de *Saccharomyces cerevisiae*.

9. Une cassette d'expression selon l'une des revendications 1 à 8, dans laquelle le fragment d'ADN code pour une protéine P30 comprenant au moins une mutation ; ladite mutation étant **caractérisée par** la présence d'un résidu acide aminé différent du résidu naturel en position 241 et/ou 243 de la séquence telle que montrée dans l'identificateur de séquence NO: 1, à la condition toutefois que ladite mutation soit **caractérisée par** un résidu autre qu'une thréonine en position 243.

10. Une cassette d'expression selon la revendication 9, **caractérisée en ce que** le résidu acide aminé en position 241 est un résidu glutamine.

11. Une cassette d'expression selon l'une des revendications 1 à 10, **caractérisée en ce que** les éléments nécessaires à l'expression dudit fragment d'ADN comprennent notamment une région promotrice fonctionnelle dans ladite cellule issue d'un organisme eucaryote non mammifère.

12. Une cassette d'expression selon la revendication 11, **caractérisée en ce que** la région promotrice est sélectionnée parmi le groupe constitué par les régions promotrices issues des gènes *PGK* de *Saccharomyces cerevisiae, adh* et *pho4* de *Schizosaccharomyces pombe* et p12,5K et p39K de baculovirus.

13. Un vecteur comprenant une cassette d'expression selon l'une des revendications 1 à 12.

14. Une cellule issue d'un organisme eucaryote non-mammifère comprenant une cassette d'expression selon l'une des revendications 1 à 12 ou un vecteur selon la revendication 13.

15. Un champignon unicellulaire ou une levure selon la revendication 14.

16. Une levure selon la revendication 15, sélectionnée parmi le groupe constitué de *Saccharomyces cerevisiae, Schizosaccharomyces pombe , Schizosaccharomyces malidevorans, Schizosaccharomyces sloofiae,* *Schizosaccharomyces octosporus* et *Hasegawaea japonicus*.

17. Une protéine P30 amputée de toute la région C-terminale hydrophobe produite par une cassette d'expression selon l'une des revendications 1 à 12, un vecteur selon la revendication 13 ou une cellule issue d'un organisme eucaryote non-mammifère selon l'une des revendications 14 à 16.

18. Un procédé de préparation d'une protéine P30 selon la revendication 17, selon lequel :
(i) on cultive dans des conditions appropriées une cellule issue d'un organisme eucaryote non-mammifère selon l'une des revendications 14 à 16 ; et
(ii) on récupère ladite protéine sécrétée de ladite cellule issue d'un organisme eucaryote non-mammifère.

19. Un réactif pour la détection et/ou la surveillance d'une infection par *Toxoplasma gondii,* **caractérisé en ce qu'**il comprend à titre de substance réactive une protéine selon la revendication 17.

20. Procédé de détection d'anticorps anti-toxoplasme dans un échantillon biologique, tel qu'un échantillon de sang, d'un individu ou d'un animal susceptible d'être ou d'avoir été infecté par *Toxoplasma gondii,* **caractérisé en ce qu'**il comprend au moins les étapes suivantes:
- on prépare un mélange comprenant :
i) un réactif selon la revendication 19, qui est ou qui sera immobilisé sur un support solide,
ii) l'échantillon,
iii) une anti-immunoglobuline marquée;
- on incube le mélange pendant un temps prédeterminé;
- on sépare la phase solide de la phase liquide; et
- on met en évidence la présence éventuelle d'anticorps anti-toxoplasme en mesurant le degré de marquage dans la phase solide.

21. Procédé selon la revendication 20, **caractérisé en ce que** :
- on prépare un mélange comprenant :
i) le réactif immobilisé sur le support solide, et
ii) l'échantillon;
- on incube le mélange pendant un temps prédéterminé permettant la formation d'un complexe immun immobilisé sur le support solide;
- on ajoute une anti-immunoglobuline marquée dans des conditions d'incubation appropriées permettant sa réaction avec le complexe immun immobilisé;
- on sépare la phase solide de la phase liquide; et
- on met en évidence la présence éventuelle d'anticorps anti-toxoplasme en mesurant le degré de marquage dans la phase solide.

22. Anticorps monoclonaux ou polyclonaux **caractérisés en ce qu'**ils sont obtenus par réaction immunologique d'un organisme humain ou animal à un agent immunogène constitués par une protéine telle que définie dans la revendication 17.

23. Réactif de détection de la présence de *Toxoplasma gondii,* **caractérisé en ce qu'**il comprend à titre de substance réactive un anticorps, selon la revendication 22, marqué.

24. Procédé de détection d'anticorps anti-toxoplasme dans un échantillon biologique, tel qu'un échantillon de sang, d'un individu ou d'un animal susceptible d'être ou d'avoir été infecté par *Toxoplasme gondii* **caractérisé en ce qu'**il comprend les étapes suivantes :
- on prépare un mélange comprenant :
i) un premier réactif, selon la revendication 19, qui est immobilisé sur un support solide,
ii) l'échantillon,
iii) un second réactif marqué, selon la revendication 23 ;
- on incube le mélange pendant un temps prédéterminé ;
- on sépare la phase solide de la phase liquide ; et
- on met en évidence la présence éventuelle d'anticorps anti-toxoplasme en mesurant le degré de marquage dans la phase solide.

25. Procédé de détection d'anticorps anti-toxoplasme dans un échantillon biologique, tel qu'un échantillon de sang, d'un individu ou d'un animal susceptible d'être ou d'avoir été infecté par *Toxoplasme gondii* **caractérisé en ce qu'**il comprend au moins les étapes suivantes :
- on prépare un mélange comprenant :
i) une anti-immunoglobuline qui est ou qui sera fixée sur un support solide;
ii) l'échantillon;
iii) un réactif selon la revendication 19, marqué;
- on incube le mélange pendant un temps prédéterminé;
- on sépare la phase liquide de la phase solide; et
- on met en évidence la présence éventuelles d'anticorps anti-toxoplasme en mesurant le degré de marquage dans la phase solide.

26. Procédé selon la revendication 25, **caractérisé en ce que**:
- on prépare un mélange comprenant :
i) une anti-immunoglobuline fixée sur le support solide;
ii) l'échantillon;
- on incube le mélange pendant un temps prédéterminé permettant la formation d'un complexe immun immobilisé sur le support solide;
- on sépare la phase liquide de la phase solide;
- on ajoute le réactif selon la revendication 19, marqué; et
- on met en évidence la présence éventuelles d'anticorps anti-toxoplasme en mesurant le degré de marquage dans la phase solide.

27. Procédé de détection de la protéine P30 de *Toxoplasma gondii* dans un échantillon biologique, tel qu'un prélèvement tissulaire, d'un individu ou d'un animal susceptible d'être ou d'avoir été infecté par *Toxoplasma gondii,* **caractérisé en ce qu'**on met en contact l'échantillon et un réactif selon la revendication 23 dans des conditions appropriées permettant une éventuelle réaction immunologique, et on détecte la présence éventuelle d'un complexe immun formé avec ledit réactif marqué en mesurant le degré de marquage dans l'échantillon biologique.

28. Une composition pharmaceutique destinée au traitement ou à la prévention d'une infection par *Toxoplasma gondii* chez un individu ou un animal, comprenant une quantité thérapeutiquement efficace d'une cassette d'expression selon l'une des revendications 1 à 12, d'un vecteur selon la revendication 13, d'une cellule issue d'un organisme eucaryote non-mammifère selon l'une des revendications 14 à 16 ou d'une protéine P30 selon la revendication 17 ou préparée selon un procédé selon la revendication 18.

29. Une composition immunothérapeutique active, notamment préparation vaccinale, **caractérise ce qu'**elle comprend à titre de principe actif, une protéine selon la revendication 17, le principe actif étant éventuellement conjugué avec un support immunologique approprié, et éventuellement un excipient pharmaceutiquement acceptable.

## Patentansprüche

1. Expressionskassette, welche in einer von einem eukaryotischen Nicht-Säugetier-Organismus stammenden Zelle funktionsfähig ist, wobei die Kassette ein ein Protein P30 von *Toxoplasma gondii* kodierendes DNA-Fragment, welches unter die Kontrolle der für dessen Expression erforderlichen Elemente gestellt ist, exprimiert, wobei das Protein P30 aus der von einem eukaryotischen Organismus stammenden Zelle sekretiert wird und durch anti-Toxoplasmen-Antiseren erkannt wird, **dadurch gekennzeichnet, dass** das DNA-Fragment ein Protein P30 kodiert, welches um die gesamte C-terminale hydrophobe Region verkürzt ist.

2. Expressionskassette nach Anspruch 1, **dadurch gekennzeichnet, dass** sie in einer Insektenzelle funktionsfähig ist.

3. Expressionskassette nach Anspruch 1, **dadurch gekennzeichnet, dass** sie in einer von einem niederen eukaryotischen Organismus stammenden Zelle funktionsfähig ist.

4. Expressionskassette nach Anspruch 3, **dadurch gekennzeichnet, dass** die von einem niederen eukaryotischen Organismus stammende Zelle eine Hefe oder ein Pilz ist.

5. Expressionskassette nach Anspruch 4, **dadurch gekennzeichnet, dass** die von einem niederen eukaryotischen Organismus stammende Zelle aus der Gruppe, gebildet aus *Saccharomyces cerevisiae, Schizosaccharomyces pombe, Schizosaccharomyces malidevorans, Schizosaccharomyces sloofiae, Schizosaccharomyces octosporus* und *Hasegawaea japonicus,* ausgewählt wird.

6. Expressionskassette nach einem der Ansprüche 1 bis 5, **dadurch gekennzeichnet, dass** das DNA-Fragment ein Protein P30 mit der Sequenz, wie sie in der Sequenzbeschreibung NR: 1 beginnend mit der Aminosäure +1 und endend mit der Aminosäure +299 gezeigt ist, oder ein immunologisches Äquivalent des Proteins P30 kodiert.

7. Expressionskassette nach einem der Ansprüche 1 bis 5, **dadurch gekennzeichnet, dass** das DNA-Fragment ein Protein P30 mit der Sequenz, wie sie in der Sequenzbeschreibung NR: 1 beginnend mit der Aminosäure +31 und endend mit der Aminosäure +299 gezeigt ist, oder ein immunologisches Äquivalent des Proteins P30 kodiert, wobei das DNA-Fragment außerdem eine Sequenz, welche ein heterologes Sekretionssignal kodiert, umfasst.

8. Expressionskassette nach Anspruch 7, **dadurch gekennzeichnet, dass** die ein heterologes Sekretionssignal kodierende Sequenz aus dem Gen *pho1* von *Schizosaccharomyces pombe* oder dem Gen des Sexualpheromons alpha (Mating Factor a, *MFa*) von *Saccharomyces cerevisiae* stammt.

9. Expressionskassette nach einem der Ansprüche 1 bis 8, in welcher das DNA-Fragment ein Protein P30 kodiert, welches wenigstens eine Mutation aufweist, wobei die Mutation durch die Anwesenheit eines von dem natürlichen Rest verschiedenen Aminosäurerests an Position 241 und/oder 243 der Sequenz, wie sie in der Sequenzbeschreibung NR 1 gezeigt ist, **gekennzeichnet** ist, gleichwohl mit der Maßgabe, dass die Mutation durch einen anderen Rest als Threonin an Position 243 **gekennzeichnet** ist.

10. Expressionskassette nach Anspruch 9, **dadurch gekennzeichnet, dass** der Aminosäurerest an Position 241 ein Glutaminrest ist.

11. Expressionskassette nach einem der Ansprüche 1 bis 10, **dadurch gekennzeichnet, dass** die für die Expression des DNA-Fragments erforderlichen Elemente insbesondere eine Promotorregion, welche in der von einem eukaryotischen Nicht-Säugetier-Organismus stammenden Zelle funktionsfähig ist, umfassen.

12. Expressionskassette nach Anspruch 11, **dadurch gekennzeichnet, dass** die Promotorregion in der Gruppe, welche aus den von den Genen *PGK* von *Saccharomyces cerevisiae,* *adh* und *pho-4* von *Schizosaccharomyces pombe* und p12,5K und p39K von Baculovirus stammenden Promotorregionen gebildet wird, ausgewählt wird.

13. Vektor, welcher eine Expressionskassette nach einem der Ansprüche 1 bis 12 umfasst.

14. Von einem eukaryotischen Nicht-Säugetier-Organismus stammende Zelle, welche eine Expressionskassette nach einem der Ansprüche 1 bis 12 oder einen Vektor nach Anspruch 13 umfasst.

15. Einzelliger Pilz oder eine Hefe nach Anspruch 14.

16. Hefe nach Anspruch 15, welche in der Gruppe, gebildet aus *Saccharomyces cerevisiae, Schizosaccharomyces pombe, Schizosaccharomyces malidevorans, Schizosaccharomyces sloofiae, Schizosaccharomyces octosporus* und *Hasegawaea japonicus,* ausgewählt wird.

17. Um die gesamte C-terminale hydrophobe Region verkürztes Protein P30, welches durch eine Expressionskassette nach einem der Ansprüche 1 bis 12, einen Vektor nach Anspruch 13 oder eine von einem eukaryotischen Nicht-Säugetier-Organismus stammende Zelle nach einem der Ansprüche 14 bis 16 exprimiert wird.

18. Verfahren zur Herstellung eines Proteins P30 nach Anspruch 17, gemäß welchem:
(i) man unter geeigneten Bedingungen eine von einem eukaryotischen Nicht-Säugetier-Organismus stammende Zelle nach einem der Ansprüche 14 bis 16 kultiviert und
(ii) man das von der von einem eukaroytischen Nicht-Säugetier-Organismus stammenden Zelle sekretierte Protein gewinnt.

19. Reagens für den Nachweis und/oder die Überwachung einer Infektion durch *Toxoplasma gondii,* **dadurch gekennzeichnet, dass** es als reaktive Substanz ein Protein nach Anspruch 17 umfasst.

20. Verfahren zum Nachweis von Anti-Toxoplasmen-Antikörpern in einer biologischen Probe, wie einer Blutprobe, von einem Individuum oder einem Tier, welches durch *Toxoplasma gondii* infiziert werden oder infiziert worden sein könnte, **dadurch gekennzeichnet, dass** es wenigstens die folgenden Schritte umfasst:
- man stellt eine Mischung her, umfassend:
i) ein Reagens nach Anspruch 19, welches an einem festen Träger immobilisiert ist oder wird,
ii) die Probe,
iii) ein markiertes Anti-Immunglobulin;
- man inkubiert die Mischung während einer vorher festgelegten Zeit;
- man trennt die feste Phase von der flüssigen Phase ab und
- man weist das etwaige Vorhandensein von Anti-Toxoplasmen-Antikörpern nach, indem man das Markierungsausmaß in der festen Phase misst.

21. Verfahren nach Anspruch 20, **dadurch gekennzeichnet, dass**:
- man eine Mischung herstellt, umfassend:
i) das an dem festen Träger immobilisierte Reagens und
ii) die Probe;
- man inkubiert die Mischung während einer vorher festgelegten Zeit, wodurch die Bildung eines an dem festen Träger immobilisierten Immunkomplexes ermöglicht wird;
- man setzt ein markiertes Anti-Immunglobulin unter geeigneten Inkubationsbedingungen, welche dessen Reaktion mit dem immobilisierten Immunkomplex ermöglichen, zu;
- man trennt die feste Phase von der flüssigen Phase ab und
- man weist das etwaige Vorhandensein von Anti-Toxoplasmen-Antikörpern nach, indem man das Markierungsausmaß in der festen Phase misst.

22. Monoklonale oder polyklonale Antikörper, **dadurch gekennzeichnet, dass** sie erhalten werden durch immunologische Reaktion eines menschlichen oder tierischen Organismus auf ein immunogenes Agens, gebildet durch ein Protein wie in Anspruch 17 definiert.

23. Reagens zum Nachweis des Vorhandenseins von *Toxoplasma gondii,* **dadurch gekennzeichnet, dass** es als reaktive Substanz einen Antikörper nach Anspruch 22, welcher markiert ist, umfasst.

24. Verfahren zum Nachweis von Anti-Toxoplasmen-Antikörpern in einer biologischen Probe, wie einer Blutprobe, von einem Individuum oder einem Tier, welches durch *Toxoplasma gondii* infiziert werden oder infiziert worden sein könnte, **dadurch gekennzeichnet, dass** es die folgenden Schritte umfasst:
- man stellt eine Mischung her, umfassend:
i) ein erstes Reagens nach Anspruch 19, welches an einem festen Träger immobilisiert ist,
ii) die Probe,
iii) ein zweites markiertes Reagens nach Anspruch 23;
- man inkubiert die Mischung während einer vorher festgelegten Zeit;
- man trennt die feste Phase von der flüssigen Phase ab und
- man weist das etwaige Vorhandensein von Anti-Toxoplasmen-Antikörpern nach, indem man das Markierungsausmaß in der festen Phase misst.

25. Verfahren zum Nachweis von Anti-Toxoplasmen-Antikörpern in einer biologischen Probe, wie einer Blutprobe, von einem Individuum oder einem Tier, welches durch *Toxoplasma gondii* infiziert werden oder infiziert worden sein könnte, **dadurch gekennzeichnet, dass** es wenigstens die folgenden Schritte umfasst:
- man stellt eine Mischung her, umfassend:
i) ein Anti-Immunglobulin, welches an einem festen Träger immobilisiert ist oder wird,
ii) die Probe,
iii) ein Reagens nach Anspruch 19, welches markiert ist;
- man inkubiert die Mischung während einer vorher festgelegten Zeit;
- man trennt die flüssige Phase von der festen Phase ab und
- man weist das etwaige Vorhandensein von Anti-Toxoplasmen-Antikörpern nach, indem man das Markierungsausmaß in der festen Phase misst.

26. Verfahren nach Anspruch 25, **dadurch gekennzeichnet, dass**:
- man eine Mischung herstellt, umfassend:
i) ein an dem festen Träger fixiertes Anti-Immunglobulin;
ii) die Probe;
- man inkubiert die Mischung während einer vorher festgelegten Zeit, wodurch die Bildung eines an dem festen Träger immobilisierten Immunkomplexes ermöglicht wird;
- man trennt die flüssige Phase von der festen Phase ab;
- man setzt das Reagens nach Anspruch 19, welches markiert ist, zu und
- man weist das etwaige Vorhandensein von Anti-Toxoplasmen-Antikörpern nach, indem man das Markierungsausmaß in der festen Phase misst.

27. Verfahren zum Nachweis des Proteins P30 von *Toxoplasma gondii* in einer biologischen Probe, wie einer Gewebeprobe, von einem Individuum oder einem Tier, welches durch *Toxoplasma gondii* infiziert werden oder infiziert worden sein könnte, **dadurch gekennzeichnet, dass** man die Probe und ein Reagens nach Anspruch 23 miteinander in Kontakt bringt unter geeigneten Bedingungen, welche eine gegebenenfalls erfolgende immunologische Reaktion erlauben, und man das etwaige Vorhandensein eines mit dem markierten Reagens gebildeten Immunkomplexes nachweist, indem man das Markierungsausmaß in der biologischen Probe misst.

28. Pharmazeutische Zusammensetzung, welche für die Behandlung oder die Verhütung einer Infektion durch *Toxoplasma gondii* bei einem Individuum oder einem Tier bestimmt ist, welche eine therapeutisch wirksame Menge einer Expressionskassette nach einem der Ansprüche 1 bis 12, eines Vektors nach Anspruch 13, einer von einem eukaryotischen Nicht-Säugetier-Organismus stammenden Zelle nach einem der Ansprüche 14 bis 16 oder eines Proteins P30 nach Anspruch 17 oder hergestellt gemäß einem Verfahren nach Anspruch 18 umfasst.

29. Immuntherapeutische aktive Zusammensetzung, insbesondere Impfzubereitung, **dadurch gekennzeichnet, dass** sie als Wirkstoff ein Protein nach Anspruch 17, wobei der Wirkstoff gegebenenfalls mit einem geeigneten immunologischen Träger konjugiert ist, und gegebenenfalls ein pharmazeutisch annehmbares Vehikel umfasst.

## Claims

1. Expression cassette which is functional in a cell derived from a nonmammalian eukaryotic organism, the said cassette expressing a DNA fragment encoding a *Toxoplasma gondii* P30 protein, placed under the control of the elements necessary for its expression, the said P30 protein being secreted from the said cell derived from a eukaryotic organism and recognized by anti-Toxoplasma antisera, **characterized in that** the said DNA fragment encodes a P30 protein in which all or part of the hydrophobic C-terminal region has been amputated.

2. Expression cassette according to Claim 1, **characterized in that** it is functional in an insect cell.

3. Expression cassette according to Claim 1, **characterized in that** it is functional in a cell derived from a lower eukaryotic organism.

4. Expression cassette according to Claim 3, **characterized in that** the cell derived from a lower eukaryotic organism is a yeast or a fungus.

5. Expression cassette according to Claim 4, **characterized in that** the cell derived from a lower eukaryotic organism is selected from the group consisting of *Saccharomyces cerevisiae, Schizosaccharomyces pombe*, *Schizosaccharomyces malidevorans, Schizosaccharomyces sloofiae, Schizosaccharomyces octosporus* and *Hasegawaea japonicus*.

6. Expression cassette according to one of Claims 1 to 5, **characterized in that** the DNA fragment encodes a P30 protein having the sequence as shown in the sequence identifier NO: 1 starting with the amino acid +1 and ending with the amino acid +299 or an immunological equivalent of the said P30 protein.

7. Expression cassette according to one of Claims 1 to 5, **characterized in that** the DNA fragment encodes a P30 protein having the sequence as shown in the sequence identifier NO: 1 starting with the amino acid +31 and ending with the amino acid +299 or an immunological equivalent of the said P30 protein, the said DNA fragment comprising, in addition, a sequence encoding a heterologous secretory signal.

8. Expression cassette according to Claim 7, **characterized in that** the said sequence encoding a heterologous secretory signal is derived from the *Schizosaccharomyces pombe pho1* gene or from the *Saccharomyces cerevisiae* alpha sex pheromone (Mating Factor *α, MFα*) gene.

9. Expression cassette according to one of Claims 1 to 8, in which the DNA fragment encodes a P30 protein comprising at least one mutation, the said mutation being **characterized by** the presence of an amino acid residue different from the natural residue in position 241 and/or 243 of the sequence as shown in the sequence identifier NO: 1, provided however that the said mutation is **characterized by** a residue other than a threonine in position 243.

10. Expression cassette according to Claim 9, **characterized in that** the amino acid residue in position 241 is a glutamine residue.

11. Expression cassette according to one of Claims 1 to 10, **characterized in that** the elements necessary for the expression of the said DNA fragment comprise especially a promoter region which is functional in the said cell derived from a nonmammalian eukaryotic organism.

12. Expression cassette according to Claim 11, **characterized in that** the promoter region is selected from the group consisting of the promoter regions derived from the genes *PGK* of *Saccharomyces cerevisiae,* *adh* and *pho4* of *Schizosaccharomyces pombe* and p12,5K and p39K of baculovirus.

13. Vector comprising an expression cassette according to one of Claims 1 to 12.

14. Cell derived from a nonmammalian eukaryotic organism comprising an expression cassette according to one of Claims 1 to 12 or a vector according to Claim 13.

15. Unicellular fungus or yeast according to Claim 14.

16. Yeast according to Claim 15, which is selected from the group consisting of *Saccharomyces cerevisiae, Schizosaccharomyces pombe, Schizosaccharomyces malidevorans, schizosaccharomyces sloofiae, Schizosaccharomyces octosporus* and *Hasegawaea japonicus*.

17. P30 protein in which all or part of the hydrophobic C-terminal region has been amputated, which is produced by an expression cassette according to one of Claims 1 to 12, a vector according to Claim 13 or a cell derived from a nonmammalian eukaryotic organism according to one of Claims 14 to 16.

18. Process for the preparation of a P30 protein according to Claim 17, according to which:
(i) a cell derived from a nonmammalian eukaryotic organism according to one of Claims 14 to 16 is cultured under appropriate conditions; and
(ii) the said protein secreted from the said cell derived from a nonmammalian eukaryotic organism is recovered.

19. Reagent for the detection and/or the monitoring of a *Toxoplasma gondii* infection, **characterized in that** it comprises, as reactive substance, a protein according to Claim 17.

20. Process for the detection of anti-Toxoplasma antibodies in a biological sample, such as a blood sample, from an individual or from an animal likely to be or to have been infected by *Toxoplasma gondii,* **characterized in that** it comprises at least the following steps:
- a mixture is prepared comprising:
i) a reagent according to Claim 19 which is or which will be immobilized on a solid support,
ii) the sample,
iii) a labelled anti-immunoglobulin;
- the mixture is incubated for a predetermined time;
- the solid phase is separated from the liquid phase; and
- the possible presence of anti-Toxoplasma antibodies is revealed by measuring the level of labelling in the solid phase.

21. Process according to Claim 20, **characterized in that**:
- a mixture is prepared comprising:
i) the reagent immobilized on the solid support, and
ii) the sample;
- the mixture is incubated for a predetermined time which allows the formation of an immune complex immobilized on the solid support;
- an anti-immunoglobulin labelled under appropriate incubating conditions allowing its reaction with the immobilized immune complex is added;
- the solid phase is separated from the liquid phase; and
- the possible presence of anti-Toxoplasma antibodies is revealed by measuring the level of labelling in the solid phase.

22. Monoclonal or polyclonal antibodies obtained by immunological reaction of a human or animal organism to an immunogenic agent consisting of a protein as defined in Claim 17.

23. Reagent for detecting the presence of *Toxoplasma gondii*, **characterized in that** it comprises, as reactive substance, an antibody according to Claim 22 which is labelled.

24. Process for the detection of anti-Toxoplasma antibodies in a biological sample, such as a blood sample, from an individual or an animal likely to be or to have been infected by *Toxoplasma gondii,* **characterized in that** it comprises the following steps:
- a mixture is prepared comprising:
i) a first reagent, according to Claim 19, which is immobilized on a solid support,
ii) the sample,
iii) a second, labelled, reagent, according to Claim 23;
- the mixture is incubated for a predetermined time;
- the solid phase is separated from the liquid phase; and
- the possible presence of anti-Toxoplasma antibodies is revealed by measuring the level of labelling in the solid phase.

25. Process for the detection of anti-Toxoplasma antibodies in a biological sample, such as a blood sample, from an individual or an animal likely to be or to have been infected by *Toxoplasma gondii,* **characterized in that** it comprises at least the following steps:
- a mixture is prepared comprising:
i) an anti-immunoglobulin which is or which will be attached onto a solid support;
ii) the sample;
iii) a reagent according to Claim 19, which is labelled;
- the mixture is incubated for a predetermined time;
- the liquid phase is separated from the solid phase; and
- the possible presence of anti-Toxoplasma antibodies is revealed by measuring the level of labelling in the solid phase.

26. Process according to Claim 25, **characterized in that**:
- a mixture is prepared comprising:
i) an anti-immunoglobulin attached onto the solid support;
ii) the sample;
- the mixture is incubated for a predetermined time allowing the formation of an immune complex immobilized on the solid support;
- the liquid phase is separated from the solid phase;
- the reagent according to Claim 19, which is labelled, is added; and
- the possible presence of anti-Toxoplasma antibodies is revealed by measuring the level of labelling in the solid phase.

27. Process for the detection of the *Toxoplasma gondii* P30 protein in a biological sample, such as a tissue sample, from an individual or an animal likely to be or to have been infected by *Toxoplasma gondii,* **characterized in that** the sample and a reagent according to Claim 23 are brought into contact under appropriate conditions which allow a possible immunological reaction, and the possible presence of an immune complex formed with the said labelled reagent is detected by measuring the level of labelling in the biological sample.

28. Pharmaceutical composition intended for the treatment or for the prevention of a *Toxoplasma gondii* infection in an individual or an animal, comprising a therapeutically effective quantity of an expression cassette according to one of Claims 1 to 12, a vector according to Claim 13, a cell derived from a nonmammalian eukaryotic organism according to one of Claims 14 to 16 or a P30 protein according to Claim 17 or prepared according to a process according to Claim 18.

29. Active immunotherapeutic composition, especially vaccinal preparation, **characterized in that** it comprises, as active ingredient, a protein according to Claim 17, the active ingredient being optionally conjugated with an appropriate immunological support, and optionally a pharmaceutically acceptable excipient.
